(19) Europäisches Patentamt
European Patent Office
Office européen des brevets

(11) Publication number: **0 002 615**
**B2**

# (12) NEW EUROPEAN PATENT SPECIFICATION

(45) Date of publication of the new patent specification: **17.01.90**

(21) Application number: **78300831.1**

(22) Date of filing: **15.12.78**

(51) Int. Cl.⁴: **C 07 D 493/22,** A 01 N 43/22, A 61 K 31/335 // C07F7/18, C12P17/08, C07H17/08 ,(C07D493/22, 313:00, 311:00, 311:00, 307:00)

## (54) 13-Halo and 13-deoxy derivatives of modified milbemycin compounds and their preparation.

(30) Priority: **19.12.77 US 861810**
**19.12.77 US 861919**

(43) Date of publication of application:
**27.06.79 Bulletin 79/13**

(45) Publication of the grant of the patent:
**05.10.83 Bulletin 83/40**

(45) Mention of the opposition decision:
**17.01.90 Bulletin 90/03**

(84) Designated Contracting States:
**BE DE FR GB NL**

(56) References cited:
**DE-A-2 717 040**
**FR-A-2 187 778**
**US-A-4 093 629**
**US-A-4 160 083**
**US-A-4 160 861**
**US-A-4 161 583**
**US-A-4 172 940**

**Antimicrobial Agents and Chemotherapy, Vol.15, No.3, Mar.1979, pp.361-367**

**Chemical Abstracts, Vol.88, No.15, 10.04.1978, Abstr.No.103351m,**

**18th Interscience Conference on Antimicrobial Agents and Chemotherapy,1-4 Oct.1978,Atlanta, Georgia, abstracts 462-465**

(73) Proprietor: **MERCK & CO. INC.**
**126, East Lincoln Avenue P.O. Box 2000**
**Rahway New Jersey 07065-0900 (US)**

(72) Inventor: **Chabala, John Clifford**
**530 Summit Avenue**
**Westfield New Jersey 07090 (US)**
Inventor: **Fisher, Michael Herbert**
**1140 Concord Drive**
**Bridgewater New Jersey 08807 (US)**
Inventor: **Mrozik, Helmut Hugo**
**159 Idlebrook Lane**
**Matawan New Jersey 07747 (US)**

(74) Representative: **Crampton, Keith John Allen**
**D YOUNG & CO 10 Staple Inn**
**London WC1V 7RD (GB)**

(56) References cited: (continuation)
**Journal of Helminthology, 52, 1978, LYNDIA S. BLAIR , WILLIAM C. CAMPBELL, "Efficacy of Avermectins against Ancylostoma caninum in dogs", pp.305-307**

**The file contains technical information submitted after the application was filed and not included in this specification**

**EP 0 002 615 B2**

LIBERGRAF, STOCKHOLM 1990

## Description

### 13-Halo and 13-Deoxy derivatives of modified milbemycin compounds and their preparation

This invention is concerned with derivatives of C-076 compounds. C-076 is a series of macrolides with potent antiparasitic activity, and is closely related structurally to the milbemycin compounds disclosed in US Patent No 4 093 629 and French Patent No 2 187 778, certain of which have the formula

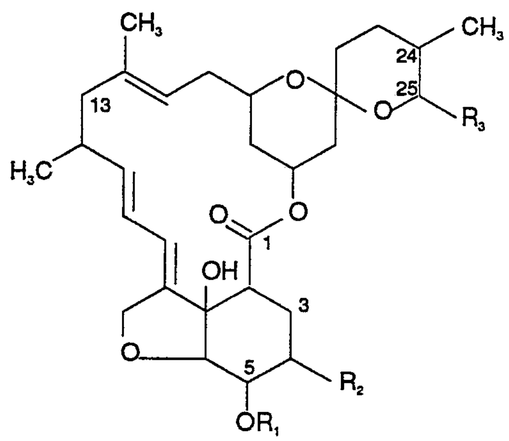

in which $R_1$ is hydrogen or methyl, $R_2$ is methyl or $-CH_2OOC$

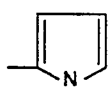

and $R_3$ is methyl or ethyl, subject to certain provisos. The compounds are isolated from the fermentation broth of *Streptomyces avermitilis* and the morphological characteristics of the microorganism as well as the methods used to isolate the C-076 compounds are described below and in German specification DE-A-2 717 040.

The present invention is more particularly concerned with C-076 derivatives that are unsubstituted or halogenated at the 13-position and in which various other positions of the C-076 compounds may be substituted, with preparation of such compounds, and with methods and compositions using such compounds as the active ingredient in the treatment of parasitic infections and infestations.

The C-076 compounds, which are the starting materials for the preparation of the compounds of the present invention, are described by the following structural formula:

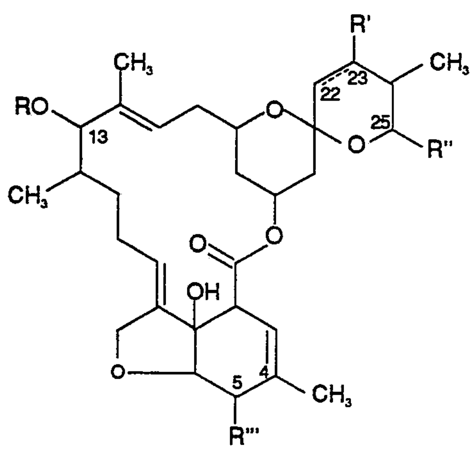

where R is the α-*L*-oleandrosyl-α-*L*-oleandrose group, which has the formula:

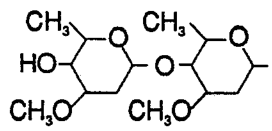

the broken line indicates a single or double bond;

R' is hydroxy and is present only when the broken line indicates a single bond;
R" is *n*-propyl or *sec*-butyl; and
R‴ is methoxy or hydroxy.

With reference to the foregoing structural formula, the individual C-076 compounds are identified as follows:

| C-076 | $R^1$ | $R^{11}$ | $R^{111}$ |
|---|---|---|---|
| Ala | Double bond | *sec*-butyl | $-OCH_3$ |
| Alb | Double bond | *n*-propyl | $-OCH_3$ |
| A2a | -OH | *sec*-butyl | $-OCH_3$ |
| A2b | -OH | *n*-propyl | $-OCH_3$ |
| B1a | Double bond | *sec*-butyl | -OH |
| B1b | Double bond | *n*-propyl | -OH |
| B2a | -OH | *sec*-butyl | -OH |
| B2b | -OH | *n*-propyl | -OH |

The formula of the *b* compound has been found to be incorrect. The file contained technical information submitted after the application was filed and not included in this specification.

The compounds of the present invention are derived from the above C-076 compounds by removing the α-*L*-oleandrosyl-α-*L*-oleandrose group and also the hydroxy group at the 13-position that remains after the disaccharide is removed. In addition, other derivatization of the 13-deoxy C-076 compounds is possible, such as acylation of one or more of the available hydroxy groups, reduction of the 22,23-double bond, alkylation of the hydroxy groups, substitution of an alkylthio group at the 23-position, and oxidized variations thereof, as well as the 13-halogenated compounds, which are intermediates in the preparation of the 13-deoxy compounds.

The compounds of the present invention are obtainable by the reaction of one of C-076 A1a, C-076 A2a, C-076 B1a, C-076 B2a, C-076 B1b, C-076 A2b, C-076 B1b and C-076 B2b, as follows:

(a) removing the α-*L*-oleandrosyl-α-*L*-oleandrose group by hydrolysis;
(b) replacing the 13-hydroxy group resulting from step (a) with a 13-halo group by reaction with a sufficiently reactive benzenesulfonyl halide in the presence of a base;
(c) optionally removing the 13-halo group with a selective reducing agent;
(d) optionally reducing the 22,23-double bond on the A1a, B1a, A1b and B1b compounds to a single bond by hydrogenation using a solvent and a catalyst of the formula $[(R_3)_3P]_3RhX$ where $R_5$ is a loweralkyl, phenyl, or loweralkyl-substituted phenyl and X is a halogen;
(e) optionally $C_{2-6}$ alkanoylating the 5- or 23- hydroxy group of the A2a, B1a, A2b or B1b compound or on one or both of the 5- and 23- hydroxy groups in the B2a or B2b compound;
(f) optionally preparing a 23-($C_{1-6}$ alkoxy) or 23-($C_{1-6}$ alkylthio) derivative of the A1a, B1a, A1b or B1b type compounds by reaction with a $C_{1-6}$ alkanol or $C_{1-6}$ alkylthiol in the presence of an acid; and
(g) optionally oxidizing a 23-($C_{1-6}$ alkylthio) derivative prepared in step (f) to a $C_{1-6}$ alkylsulphinyl or a $C_{1-6}$ alkylsulphonyl group.

The *a* compounds of the present invention have the following structural formula:

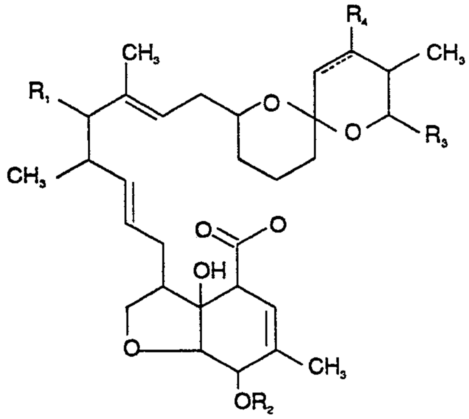

where the broken line indicates a single or double bond;

$R_1$ is hydrogen or halogen;
$R_2$ is hydrogen, methyl or loweralkanoyl;
$R_3$ is *sec*-butyl; and
$R_4$ is present only when the broken line indicates a single bond and represents hydrogen, hydroxy, loweralkanoyloxy, loweralkylthio, loweralkylsulfinyl, loweralkylsulfonyl or loweralkoxy.

In the present specification the term "loweralkoxy" means those alkoxy groups containing from 1 to 6 carbon atoms in either a straight or branched configuration. Examples are methoxy, ethoxy, propoxy, *iso*-propoxy, butoxy, *tert*-butoxy, pentoxy and hexoxy.

The terms "loweralkylthio", "loweralkylsulfinyl" and "loweralkylsulfonyl" means those groups that contain an alkyl group of from 1 to 6 carbon atoms in either a straight or branched chain. Examples are methyl, ethyl, propyl, *iso*-propyl, butyl, *sec*-butyl, pentyl and hexyl.

The terms "loweralkanoyl" and "loweralkanoyloxy" mean those alkanoyl and alkanoyloxy groups that contain from 2 to 6 carbon atoms in either a straight or branched configuration. Examples are acetyl, propionyl, butyryl, pentanoyl, hexanoyl and pivaloyl.

The term "halogen" or "halo" means fluorine, chlorine, bromine or iodine.

The compounds of the invention may be prepared by a series of reactions which converts the C-076 starting materials from a 13-disaccharide series of compounds to the aglycone compound (13- position is hydroxy) followed by the conversion of the 13-hydroxy group to the 13-halogen and 13-deoxy groups. In addition, the $R_2$ and $R_4$ substituent groups and the 22,23-unsaturation may be reacted to form other substituents.

As is readily apparent from an analysis of the structure of the C-076 starting materials, there are five unsaturations in the 1-series of compounds. The process of this invention involves reducing the 22,23-double bond while not affecting the remaining four unsaturations or any other functional group present on the molecule. It is necessary to select a specific catalyst for the hydrogenation, viz. one that will selectively hydrogenate the least hindered from among a series of unsaturations and the catalyst used has the formula $[(R_5)_3P]_3RhX$ where $R_5$ and X are defined above. In the preferred catalyst $R_5$ is phenyl and X is chlorine, that is the compounds is tris(triphenylphosphine)rhodium(I)chloride, which is also known as Wilkinson's homogeneous catalyst.

The reaction is carried out using a catalytic amount of the catalyst. The amount of catalyst is not critical and from 0.05 to 0.5 mole of the catalyst for each mole of starting material has been successfully used. Molar ratios in the range of 0.25 : 1 to 0.40 : 1 are preferred.

The hydrogenation is carried out in a hydrogen atmosphere which may be either at atmospheric pressure or up to about 400,000 Pa (4 atmospheres) pressure in a standard laboratory hydrogenation apparatus. A solvent is employed to dissolve both the starting materials and the catalyst. Preferred solvents are hydrocarbon solvents such as benzene, toluene, petroleum ether and other alkane hydrocarbons. The reaction is complete when the calculated amount of hydrogen has been taken up by the reaction. This will generally require from 16 to 48 hours. The reaction may be carried out at from room temperature to 75°C, however, room temperature is preferred. The hydrogenation products are isolated and purified by techniques known to those skilled in the art.

Other reactions may be carried out on the C-076 starting materials or on the hydrogenated products to prepare other compounds of this invention. While it is possible to complete other reactions on the C-076 starting material and have the hydrogenation step as the final reaction, it is preferred to carry out the hydrogenation step before the reactions at the 5- or 13-position. Because the 22,23-double bond is somewhat susceptible to electrophilic addition, reaction conditions for removing the sugar groups or acylating the hydroxy groups must be

carefully controlled if the 22,23-double bond is present. If the 22,23-double bond is hydrogenated first, the other reactions are rendered more facile.

The compounds acylated at the 5- and 23-positions ($R_2$ or $R_4$ as loweralkanoyl) are prepared using acylation techniques in which the reaction conditions will vary, depending upon the reactivity of the hydroxy group being acylated. Where there is more than one hydroxy group to be acylated, different reaction conditions are employed to minimize the formation of mixtures.

The preferred acylation reagents employed are generally the loweralkanoyl halide, preferably the chloride, or the loweralkanoyl anhydride.

In the case of reactions carried out with the halide reagents, it is often advantageous to include in the reaction mixture a basic compound capable of reacting with and neutralizing the hydrogen halide which is liberated during the course of the reaction. Tertiary amines are preferred such as triethylamine, pyridine, 4-dimethylamino pyridine, and diisopropyl ethylamine. The basic compound is required in equimolar amounts relative to the number of moles of hydrogen halide being liberated, however excess amounts, even using the basic compound as a solvent are not detrimental.

In the case of the A1 aglycone compounds, there are no hydroxy groups which may be acylated.

The A2 compounds have a single available hydroxy group at the 23-position capable of being acylated.

The 23-acyl compound may be prepared by heating the reaction mixture at from room temperature to 100°C for from 1 to 24 hours.

The B1 compounds also have a single available hydroxy group at the 5-position. The reaction with the acylating agent is carried out in pyridine from 0°C to room temperature for from 4 to 24 hours. To recover the acylated compounds, the reaction mixture is eluted through a chromatographic column or a preparative layer chromatographic plate of alumina or silica gel and the purified compounds are readily isolated. In addition, other techniques, such as high pressure liquid chromatography, may be employed.

The B2 compounds have two hydroxy groups available for substitution: the 5- and 23-positions. The 5,23-diacyl compound may be prepared by carrying out the reaction at from room temperature to 100°C for from 1 - 24 hours. The 5-acyl compound may be prepared for carrying out the reaction at from about 0°C to room temperature for from 4 - 24 hours. To prepare the 23-acyl compound, the 5,23-diacyl compound is hydrolysed with an aqueous base such as aqueous sodium hydroxide, at about room temperature for from 1 to 24 hours. The 5-acyl group will be hydrolysed, leaving the 23-monoacyl compound.

The above described acyl compounds are isolated from the reaction mixture using techniques known to those skilled in this art.

The compounds where the 23-substituent ($R_4$) is loweralkoxy or loweralkylthio are prepared from the 1-series of compounds (the compounds with a 22,23-unsaturation). This unsaturation is more readily susceptible to electrophilic addition than the other unsaturations in the molecule, thus by monitoring reaction conditions carefully, the reaction can be made fairly specific.

The reaction is carried out in the presence of an acid and a loweralkanol or a loweralkythiol. The reaction may be carried out in an inert, aprotic solvent such as dioxane, tetrahydrofuran, ether and the like or if the alcohol or thiol is available in sufficient quantities, then said alcohol or thiol may be used in large excess and the inert solvent dispensed with. Among suitable acids are sulfuric, hydrohalic, phosphoric, trifluoroacetic, and trifluoromethanesulfonic. The preferred hydrohalic acid is hydrochloric or hydrobromic. The especially preferred acid is sulfuric acid. The acid is present in the reaction mixture at from 0.1 to 10 % by weight. The reaction is complete generally at from 0 - 50°C for from 2 to 24 hours. It is preferred to stir the reaction mixture overnight at room temperature.

Occasionally, to a small extent, there may be found some 22-addition products in the reaction mixture. This will be a minor side product, since the 23-substituent is the thermodynamically preferred compound, and the impurity is readily removed by chromatographic separation.

The 23-loweralkylthio substituent is oxidized to the 23-loweralkylsulfinyl and 23-loweralkylsufonyl group with a mild oxidizing agent. The preferred oxidizing agent is *m*-chloroperbenzoic acid and the reaction is generally carried out in a solvent inert to oxidation. Halogenated hydrocarbons such as methylene chloride or chloroform are suitable. To prepare the sulfoxide a single molar equivalent of the oxidizing agent is employed and the reaction is complete in from 5 minutes to 1 hour at from -20°C to room temperature. To prepare the sulfone two equivalents of the oxidizing agent are used and the reaction is complete in from 1 - 24 hours at from 0°C to room temperature. The products are isolated using techniques known to those skilled in this art.

The 13-position substituents ($R_1$ = halogen, hydrogen) are prepared from the C-076 starting materials as described hereinbelow. The reaction at the 13-position can generally be carried either before or after the other above described reactions.

The series of reactions at the 13-position commences with the removal of the α-*L*-oleandrosyl-α-*L*-oleandrose side chain which is found in the C-076 starting materials. This reaction produces what is identified as the "C-076 aglycone" compounds characterized by having a hydroxy group at the 13-position. The C-076 aglycone compounds are then halogenated with a suitably reactive benzenesulfonyl chloride or bromide in the presence of a base to produce the "13-deoxy-13-halo-C-076-aglycone" compounds. The halogen is then removed in a reaction with a trialkyltinhydride to produce the "13-deoxy-C-076-aglycone compounds."

The reaction conditions which are generally applicable to the preparation of C-076 aglycone involve dissolving the C-076 compound in an aqueous non-nucleophilic organic solvent miscible with water, preferably dioxane, tetrahydrofuran, dimethoxyethane, dimethyl formamide or *bis*-2-methoxy-ethyl ether in which the water

concentration is from 0.1 to 20 % by volume. Acid is added to the aqueous organic solvent to the extent of 1.0 to 10 % by volume. The reaction mixture is generally stirred at 20 - 40°C, preferably at room temperature, for from 6 to 24 hours. The products are isolated, and mixtures are separated by techniques such as column, thin layer, preparative layer and high pressure liquid chromatography, and other known techniques.

The acids which may be employed in the above process include mineral acids and organic acids such as sulfuric, hydrohalic, phosphoric, trifluoroacetic and trifluoromethanesulfonic acids. The hydrohalic acids are preferably hydrochloric or hydrobromic. The preferred acid in the above process is sulfuric acid.

A further procedure for the preparation of the aglycone compounds is applicable to all of the C-076 compounds. However, it is preferred for use on the compounds wherein the broken line indicates a single bond, since some degree of addition to the 22,23-double bond is noticed in those compounds with the 22,23-unsaturation. The procedure for the preparation of the aglycone, 1 % sulfuric acid, by volume, in methanol at from 20 - 40°C, preferably room temperature, for from 6 - 24 hours has been found to be appropriate.

The other acids listed above may also be employed for this process, at approximately the concentration employed for sulfuric acid.

The above described compounds are isolated from the reaction mixture and mixtures of compounds are separated using techniques known to those skilled in this art, and in particular the chromatographic techniques described above.

The "C-076 aglycone" thus produced is then halogenated to produce the 13-deoxy-13-halo-C-076 aglycone. The halogenation is most readily carried out in the presence of a sufficiently reactive benzenesulfonyl halide compound in the presence of a base. The presence of electron withdrawing substituents on the benzenesulfonyl halide is advantageous and *o*-nitro substitution is preferred. The reaction is carried out in a non-aprotic inert solvent such as a halogenated alkyl compound, preferably methylene chloride or chloroform. The reactants are combined slowly at an initial temperature of from -25 to 10°C to control any initial exothermic reactions and is maintained at this temperature for up to 2 hours. The reaction temperature is then raised to from about room temperature to the reflux temperature of the reaction mixture for from 10 minutes to 6 hours. It is necessary to carry out the reaction in the presence of a base, preferably an organic amine. It has been found to be preferable to employ the combination of a 4-di(loweralkyl)amino pyridine and trialkylamine. It is most preferred to employ 4-dimethylamino pyridine and diisopropylethylamine as bases for the foregoing reactions. The 13-deoxy-13-halo-C-076 aglycone compounds are isolated by procedures known to those skilled in this art.

In order to avoid unwanted side-reactions, it is important that, in those C-076 compounds with a hydroxy group at the 5-position (the B-series of compounds), and to a lesser extent, the 23-hydroxy group of the 2-series of compounds, said hydroxy groups be protected. The protecting group is ideally one which may be readily synthesized, will not be affected by the reactions to alter the 13-position substituent, and may be readily removed without affecting any other function of the molecule. One preferred type of protecting group for the C-076 type of molecule is the trisubstituted silyl group, preferably a trialkylsilyl group. One preferred example is the tert-butyldimethylsilyl group. The reaction is carried out by reacting the hydroxy compound with the appropriately substituted silyl halide, preferably the silyl chloride, in an aprotic polar solvent such as dimethylformamide. Imidazole is added as a catalyst. The reaction is complete in from 1/2 to 24 hours at from 0 - 25°C. For the 5-position hydroxy group the reaction is complete in from 1/2 to 3 hours at from 0°C to room temperature. The silylation reaction is much slower at the 23-position hydroxy group (the 2-series of compounds), then at the 5-position hydroxy group, and protection is generally not necessary. However, if it is desired to protect the 23-hydroxy group, the reaction will be complete in from 5 to 24 hours at from room temperature to 75°C. This reaction is selective to the 5- and 23-positions under the conditions above described, and very little, if any, silylation is observed at the 13-position.

The silyl group may be removed after the 13-halogenation or the reaction may be deferred until after the 13-halo group is removed. The silyl group or groups are removed by stirring the silyl compound in methanol catalysed by a catalytic amount of an acid, preferably a sulfonic acid such as *p*-toluenesulfonic acid. The reaction is complete in from 1 to 12 hours at from 0 to 50°C.

The 13-deoxy-13-halo-C-076 aglycone which may or may not have the silyl groups protecting the 5- and 23-hydroxy groups is then reduced to form the 13-deoxy-C-076 aglycone. The preferred reducing agent is one that will selectively remove the 13-halo group but will leave the remainder of the molecule untouched. One such reducing agent is a trialkyltinhydride, preferably tributyltinhydride. In addition it is preferable to include in the reaction mixture a free radical initiator since it is believed that the reaction proceeds through a free radical mechanism (not wishing to be bound by theory, however, other possible mechanisms are not excluded). Acceptable free radical initiators are varied and include peroxides, such as dibenzoyl peroxides; thiols in the presence of air; azodialkylnitriles such as azobisisobutyronitrile; ultraviolet light; heat and the like. The reaction conditions will vary depending upon the type of free radical initiator which is employed. For chemical initiators the reaction is complete in from 1 to 6 hours at from 60 - 120°C. The preferred reaction temperature is about 85°C. If heat is the initiating agent, higher temperatures are required, 100 - 200°C for from 1 - 6 hours. If ultraviolet light is employed, lower temperatures are preferred. Generally the reaction will be complete in from 1 - 6 hours at -25 to 50°C in the presence of ultraviolet light. The trialkyltinhydride reaction is generally carried out with no solvent under a blanket of nitrogen or other inert gas. The tin hydride compound is used in excess and becomes the solvent. If desired, however, an inert solvent such as benzene, toluene, xylene and the like could be employed. For obvious reasons, halogenated solvents cannot be employed. The products are isolated using procedures known to those skilled in this art.

Except for the case of the 22,23-hydrogenation reaction and the silylation reaction above mentioned, there is no requirement that the above reactions be carried out in any particular order. No conflicting reactions, save for the above exceptions, are found in the foregoing series of reactions and a reaction at one particular position will not affect any substituent groups at another position.

The novel 13-halo- and 13-deoxy-C-076 compounds of this invention have significant parasiticidal activity as anthelmintics, ectoparasiticides, insecticides and aracicides, in human and animal health and in agriculture.

The disease or group of diseases described generally as helminthiasis is due to infection of an animal host with parasitic worms known as helminths. Helminthiasis is a prevalent and serious economic problem in domesticated animals such as swine, sheep, horses, cattle, goats, dogs, cats and poultry. Among the helminths, the group of worms described as nematodes causes widespread and often times serious infection in various species of animals. The most common genera of nematodes infecting the animals referred to above are *Haemonchus, Trichostrongylus, Ostertagia, Nematodirus Cooperia, Ascaris, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris* and *Parascaris.* Certain of these, such as *Nematodirus, Cooperia,* and *Oesphagostomum* attack primarily the intestinal tract while others, such as *Haemonchus* and *Ostertagia* are more prevalent in the stomach while still others such as *Dictyocaulus* are found in the lungs. Still other parasites may be located in other tissues and organs of the body such as the heart and blood vessels, subcutaneous and lymphatic tissue and the like. The parasitic infections known as helminthiases lead to anaemia, malnutrition, weakness, weight loss, sever damage to the walls of the intestinal tract and other tissues and organs and, if left untreated, may result in death of the infected host. The 13-halo- and 13-deoxy-C-076 compounds of this invention have unexpectedly high activity against these parasites, and in addition are also active against *Dirofilaria* in dogs, *Nematospiroiodes, Syphacia,* and *Aspiciuluris* in rodents, arthropod ectoparasites of animals and birds such as ticks, mites, lice, fleas, blowfly in sheep *Lucilia sp.*, biting insects and such migrating diperous larvae as *Hypoderma sp.* in cattle, *Gastrophilus* in horses, and *Cuterebra sp.* in rodents.

The compounds are also useful against parasites which infect humans. The most common genera of parasites of the gastro-intestinal tract of man are *Ancylostoma, Necator, Ascaris, Strongyloides, Trichinella, Capillaria, Trichuris,* and *Enterobius.* Other medically important genera of parasites which are found in the blood or other tissues and organs outside the gastrointestinal tract are the filiarial worms such as *Wuchjeria, Brugia, Onchocerca* and *Loa, Dracunculus* and extra intestinal stages of the intestinal worms *Strongyloicles* and *Trichinella.* The compounds are also of value against arthropods parasitizing man, biting insects and other dipterous pests causing annoyance to man.

The compounds are also active against household pests such as the cockroach, *Blatella sp.*, clothes moth, *Tineola sp.*, carpet beetle, *Attagenus sp.*, and the housefly *Musca domestica.*

The compounds are also useful against insect pests of stored grains such as *Tribolium sp., Tenebrio sp.*, and of agricultural plants such as spider mites. (*Tetranychus sp.*), aphids, (*Acyrthiosiphon sp*)., against migratory orthopterans such as locust and immature stages of insects living on plant tissue. The compounds are useful as a nematocide for the control of soil nematodes and plant parasites such as *Meloidogyne spp.* which may be of importance in agriculture.

These compounds may be administered orally in a unit dosage form such as a capsule, bolus or tablet, or as a liquid drench where used as an anthelmintic in mammals. The drench is normally a solution, suspension or dispersion of the active ingredient usually in water together with a suspending agent such as bentonite and a wetting agent or like excipient. Generally, the drenches also contain an antifoaming agent. Drench formulations generally contain from 0.001 to 0.5 % by weight of the active compound. Preferred drench formulations may contain from 0.01 to 0.1 % by weight. The capsules and boluses comprise the active ingredient admixed with a carrier vehicle such as starch, talc, magnesium stearate, or di-calcium phosphate.

Where it is desired to administer the C-076 derivatives in a dry, solid unit dosage form, capsules, boluses or tablets containing the desired amount of active compound usually are employed. These dosage forms are prepared by intimately and uniformly mixing the active ingredient with suitable finely divided diluents, fillers, disintegrating agents and/or binders such as starch, lactose, talc magnesium stearate, and vegetable gums. Such unit dosage formulations may be varied widely with respect to their total weight and content of the antiparasitic agent depending upon factors such as the type of host animal to be treated, the severity and type of infection and the weight of the host.

When the active compound is to be administered via an animal feedstuff, it is intimately dispersed in the feed or used as a top dressing or in the form of pellets which may then be added to the finished feed or optionally fed separately. Alternatively, the antiparasitic compounds of our invention may be administered to animals parenterally, for example, by intramuscular, intratracheal, or subcutaneous injection in which event the active ingredient is dissolved or dispersed in a liquid carrier vehicle. For parenteral administration, the active material is suitably admixed with an acceptable vehicle, preferably of the vegetable oil variety such as peanut oil, or cotton seed oil. Other parenteral vehicles such as organic preparation using solketal, glycerol, formal and aqueous parenteral formulations are also used. The active 13-halo- or 13-deoxy-C-076 compound or compounds are dissolved or suspended in the parenteral formulation for administration; such formulations generally contain from 0.005 to 5 % by weight of the active compound.

Although the antiparasitic agents of this invention find their primary use in the treatment and/or prevention of helminthiasis, they are also useful in the prevention and treatment of diseases caused by other parasites, for example, arthropod parasites such as ticks, lice, fleas, mites and other biting insects in domesticated animals

and poultry. They are also effective in treatment of parasitic diseases that occur in other animals including humans. The optimum amount to be employed for best results will, of course, depend upon the particular compound employed, the species of animal to be treated and the type and severity of parasitic infection or infestation. Generally good results are obtained with our novel compounds by the oral administration of from 0.001 to 10 mg per kg of animal body weight, such a total dose being given at one time or in divided doses over a relatively short period of time such as 1 - 5 days. With the preferred compounds of the invention, excellent control of such parasites is obtained in animals by administering from 0.025 to 0.5 mg per kg of body weight in a single dose. Repeat treatments are given as required to combat re-infections and are dependent upon the species of parasite and the husbandry techniques being employed. The techniques for administering these materials to animals are known to those skilled in the veterinary field.

When the compounds described herein are administered as a component of the feed of the animals, or dissolved or suspended in the drinking water, compositions are provided in which the active compound or compounds are intimately dispersed in an inert carrier or diluent. By inert carrier is meant one that will not react with the antiparasitic agent and one that may be administered safely to animals. Preferably, a carrier for feed administration is one that is, or may be, an ingredient of the animal ration.

Suitable compositions include feed premixes or supplements in which the active ingredient is present in relatively large amounts and which are suitable for direct feeding to the animal or for addition to the feed either directly or after an intermediate dilution or blending step. Typical carriers or diluents suitable for such compositions include, for example, distillers' dried grains, corn meal, citrus meal, fermentation residues, ground oyster shells, wheat shorts, molasses solubles, corn cob meal, edible bean mill feed, soya grits, and crushed limestone. The active 13-halo- or 13-deoxy-C-076 compounds are intimately dispersed throughout the carrier by methods such as grinding, stirring milling or tumbling. Compositions containing from 0.005 to 2.0 % by weight of the active compound are particularly suitable as feed premixes. Feed supplements, which are fed directly to the animal, contain from 0.0002 to 0.3 % by weight of the active compounds.

Such supplements are added to the animal feed in an amount to give the finished feed the concentration of active compound desired for the treatment and control of parasitic diseases. Although the desired concentration of active compound will vary depending upon the factors previously mentioned as well as upon the particular C-076 derivative employed, the compounds of this invention are usually fed at concentrations of between 0.00001 to 0.002 % in the feed in order to achieve the desired antiparasitic result.

In using the compounds of this invention, the individual 13-halo- and 13-deoxy-C-076 components may be prepared and used in that form. Alternatively, mixtures of two or more of the individual 13-halo- and 13-deoxy-C-076 components may be used, as well as mixtures of the parent C-076 compounds and the compounds of this invention.

In the isolation of the C-076 compounds, which serve as starting materials for the instant processes, from the fermentation broth, the various C-076 compounds will be found to have been prepared in unequal amounts. In particular an "a" series compound will be prepared in a higher proportion than the corresponding "b" series compound. The weight ratio of "a" series to the corresponding "b" series is about 85 : 15 to 99 : 1. The differences between the "a" series and "b" series is constant throughout the the C-076 compounds and consists of an *n*-propyl group and *sec*-butyl group respectively at the 25-position. This difference, of course, does not interfere with any of the present reactions. In particular, it may not be necessary to separate the "b" components from the related "a" component. Separation of these closely related compounds is generally not practised since the "b" compound is present only in a very small percent by weight, and the structural difference has negligible effect on the reaction processes and biological activities.

The C-076 compounds of this invention are also useful in combating agricultural pests that inflict damage upon crops while they are growing or while in storage. The compounds are applied using known techniques in forms such as sprays, dusts, and emulsions, to the growing or stored crops to effect protection from such agricultural pests.

The following examples are provided in order that this invention might be more fully understood; they are not to be construed as limitative of the invention.

The 13-halo- and 13-deoxy-C76 derivatives prepared in the following examples are generally isolated as amorphous solids and not as crystalline solids. They are characterized analytically using techniques such as mass spectrometry, nuclear magnetic resonance, and the like. Being amorphous, the compounds are not characterized by sharp melting points: however, the chromatographic and analytical methods employed indicate that the compounds are pure.

**Example 1**

**23-O-t-Butyldimethylsilyl-C-076-A2a-Aglycone**

200 mg of C-076-A2a-aglycone in 2.4 ml of dry dimethylformamide is combined with 133 mg of imidazole and stirred until all the components are dissolved. 146 mg of *t*-butyldimethylsilylchloride is added and the reaction mixture stirred at room temperature for 24 hours. The reaction mixture is diluted with ether and washed five times with water. The combined water washes are extracted with ether and the combined organic layers washed again with water, followed by a single wash with saturated sodium chloride solution. The ether layer is concentrated to dryness *in vacuo* affording 340

mg of a gold colored oil. Preparative layer chromatography of the oil on two plates of silica gel eluting with a mixture of 5 % tetrahydrofuran and 5 % ethanol in methylene chloride affords 113.2 mg of 23-O-*t*-butyldimethylsilyl-C-076-A2a-aglycone, the structure of which is confirmed by mass spectrometry, and nuclear magnetic resonance.

**Example 2**

**23-O-t-Butyldimethylsilyl-13-Chloro-13-Deoxy-C-076-A2a-Aglycone**

20 mg of 23-O-*t*-butyldimethylsilyl-C-076-A2a-aglycone is combined with 0.7 ml of a methylene chloride solution containing 15 mg of 4-dimethylaminopyridine and 0.021 ml (15.5 mg) of diisopropylethylamine. The mixture is cooled in an ice bath and a solution of 0.1 ml of methylene chloride containing 20 mg of *o*-nitrobenzenesulfonylchloride is added dropwise. The reaction mixture is stirred for 45 minutes in an ice bath, and for 3 hours at room temperature. Ice chips are added to the reaction mixture and stirred. When the ice is melted ether is added to the mixture and the layers separated. The aqueous layer is again extracted with ether and the combined organic layers washed twice with water, dried over magnesium sulfate and evaporated to dryness under a stream of nitrogen affording 35 mg of a gold film. Preparative layer chromatography of the material on a single silica gel plate eluting with 5 % tetrahydrofuran and 5 % ethanol in methylene chloride affords 10.1 mg of 23-O-*t*-butyldimethylsilyl-13-chloro-13-deoxy-C-076-A2a-Aglycone, the structure of which is confirmed by mass spectrometry and 300 MHz nuclear magnetic resonance.

**Example 3**

**13-Chloro-13-Deoxy-C-076-A2a-Aglycone**

A solution of 10 mg of 23-O-*t*-butyldimethylsilyl-13-deoxy-C-076-A2a-aglycone in 1.0 ml of methanol containing 1 % *p*-toluene sulfonic acid dihydrate is stirred at room temperature for 5 hours. The reaction mixture is diluted with 25 ml of ethyl acetate, and washed with aqueous sodium bicarbonate and water. The organic layer is dried and evaporated to dryness *in vacuo* affording 13-chloro-13-deoxy-C-076-A2a-aglycone.

**Example 4**

**13-Chloro-13-Deoxy-C-076-A2a-Aglycone**

20 mg of C-076-A2a-aglycone is dissolved in 0.7 ml of methylene chloride containing 16 mg of 4-dimethylaminopyridine and 16.8 mg (0.023 ml) of diisopropylethylamine. The reaction mixture is cooled in an ice bath and 0.1 ml of methylene chloride containing 21.5 mg of *o*-nitrobenzene-sulfonylchloride is added dropwise. The reaction mixture is stirred in an ice bath for 1 hour, allowed to warm to room temperature and stirred for 4 hours. Ice is added and stirred until melted. Ether is added and the layers shaken and separated. The aqueous layer is extracted with ether and the organic layers combined, washed three times with water, dried over magnesium sulfate and evaporated to dryness under a stream of nitrogen affording 40 mg of a brown film. Preparative layer chromatography on silica gel eluting with 3 % tetrahydrofuran and 1 % ethanol in methylene chloride affords 4.7 mg of 13-chloro-13-deoxy-C-076-A2a-aglycone, which is identified by nuclear magnetic resonance and mass spectrometry.

**Example 5**

**13-Deoxy-C-076-A2a-Aglycone**

80 mg of 13-chloro-13-deoxy-C-076-A2a-aglycone is dissolved in 1.5 ml of tributyltinhydride and 20 mg of azobisisobutyronitrile is added. The reaction is heated under a blanket of nitrogen at 85°C for 3 1/2 hours, cooled and placed on a silica gel preparative layer chromatography plate and eluted with chloroform affording 110 mg of a glass. Repeated preparative layer chromatography on silica gel using methylene chloride with 2 % tetrahydrofuran and 0.07 % ethanol as eluent affords 70 mg of a white glass which is identified by mass spectrometry and nuclear magnetic resonance as 13-deoxy-C-076-A2a-aglycone.

**Example 6**

**13-deoxy-C-076-A2a-Aglycone**

Following the procedure of Example 3 using 13-deoxy-23-O-*t*-butyldimethylsilyl-C-076-A2a-aglycone in place of 23-O-*t*-butyldimethylsilyl-13-chloro-13-deoxy-C-076-A2a-aglycone, there is obtained 13-deoxy-C-076-A2a-aglycone.

**Example 7**

**5-O-*t*-Butyldimethylsilyl-C-076-B1a-Aglycone**

100 mg of C-076-B1a-aglycone is dissolved in 1.2 ml of anhydrous dimethylformamide and 46 mg of imidazole is added followed by 50 mg of *t*-butyldimethylsilylchloride. The reaction is maintained at 20°C for 30 minutes and diluted with ether. The mixture is washed with water, dried and concentrated *in vacuo* to a colorless glass. Further purification on a preparative layer chromatography plate eluting with a methylene chloride, tetrahydrofuran mixture affords purified 5-O-*t*-butyl-dimethylsilyl-C-076-B1a-aglycone.

Following the above procedure, utilizing C-076-B2a-aglycone in place of C-076-B1a-aglycone, affords 5-O-*t*-butyldimethylsilyl-C76-B2a-aglycone.

**Example 8**

**5-O-t-Butyldimethylsilyl-13-Deoxy-13-chloro-C-076-B1a-Aglycone**

Following the procedure of Example 4 utilizing 5-O-*t*-butyldimethylsilyl-C-076-B1a-aglycone in place of C-076-A2a-aglycone, there is produced 5-O-*t*-butyldimethylsilyl-13-deoxy-13-chloro-C-076-B1a-aglycone.

Following the above referenced procedure using 5-O-*t*-butyldimethylsilyl-C-076-B2a-aglycone in place of 5-O-*t*-butyldimethylsilyl-C-076-B1a-aglycone, there is obtained 5-O-*t*-butyldimethylsilyl-13-deoxy-13-chloro-C-076-B2a-aglycone.

**Example 9**

**5-O-t-Butyldimethylsilyl-13-Deoxy-C-076-B1a-Aglycone**

Following the procedure of Example 5 utilizing 5-O-*t*-butyldimethylsilyl-13-deoxy-13-chloro-C-076-B1a-aglycone in place of 13-chloro-13-deoxy-C-076-A2a-aglycone, there is produced 5-O-*t*-butyldimethylsilyl-13-deoxy-C-076-B1a-aglycone.

Following the above referenced procedure using 5-O-*t*-butyldimethylsilyl-13-deoxy-13-chloro-C-076-B2a in place of 5-O-*t*-butyldimethylsilyl-13-deoxy-13-chloro-C-076-B1a-aglycone, there is produced 5-O-*t*-butyldimethylsilyl-13-deoxy-C-076-B2a-aglycone.

**Example 10**

**13-Deoxy-C-076-B1a-Aglycone**

A solution of 13 mg of 5-O-*t*-butyldimethylsilyl-13-deoxy-C-076-B1a-aglycone in 1.0 ml of methanol containing 1 % *p*-toluenesulfonic acid dihydrate is stirred at 20°C for 3 hours. The reaction is diluted with 30 ml of ethyl acetate, washed with aqueous sodium bicarbonate solution, and then with water. The organic layer is dried and evaporated to dryness *in vacuo* to afford 13-deoxy-C-76-B1a-aglycone as a clear glass.

Following the above procedure, utilizing 5-O-*t*-butyldimethylsilyl-13-deoxy-C-076-B2a-aglycone in place of 5-O-*t*-butyldimethylsilyl-13-deoxy-C-076-B1a-aglycone, there is obtained 13-deoxy-C-076-B2a-aglycone.

If the products of Example 8 are hydrolysed according to the foregoing procedure, there will be obtained 13-chloro-13-deoxy-C-076-B1a-aglycone and 13-chloro-13-deoxy-C-076-B2a-aglycone.

**Example 11**

**13-Chloro-13-Deoxy-C-076-A1a-Aglycone**

Following the procedure of Example 4, employing C-076 A1a-aglycone in place of C-076 A2a-aglycone, there is produced 13-chloro-13-deoxy-C-076-A1a-aglycone.

**Example 12**

**13-Deoxy-C-076-A1a-Aglycone**

Following the procedure of Example 5, employing 13-chloro-13-deoxy-C-076-A1a-aglycone in place of 13-chloro-13-deoxy-C-076-A2a-aglycone, there is produced 13-deoxy-C-076- A1a-aglycone.

**Example 13**

**13-Chloro-13-Deoxy-22,23-Dihydro-C-076-A1a-Aglycone**

A solution of 8.2 mg of 22,23-dihydro-C-076-A1a-aglycone and 0.35 ml of methylene chloride containing 7.5 mg of 4-dimethylaminopyridine and 10.5 microliters of diisopropylethylamine is cooled to 0°C and treated with 10 mg of *o*-nitrobenzenesulfonylchloride. After being stirred for 1 hour at 0°C the reaction mixture is warmed to room temperature for 2 hours. The reaction mixture is quenched with ice and treated with 2 ml of ether. The layers are separated and aqueous phase washed twice with 1 ml of ether. The combined organic layers are washed twice with water, dried over sodium sulfate and evaporated to dryness *in vacuo*. The product is isolated by preparative layer chromatography on a single silica gel plate eluting with chloroform. Lyophilization of the residue affords 1.3 mg of a white powder identified by mass spectrometry and nuclear magnetic resonance as 13-chloro-13-deoxy-22,23-dihydro-C-076-A1a-aglycone.

**Example 14**

**13-Deoxy-22,23-Dihydro-C-076-A1a-Aglycone**

A solution of 1.0 mg of 13-chloro-13-deoxy-22,23-dihydro-C-076-A1a-aglycone is dissolved in 0.2 ml of tributyltinhydride containing 0.2 mg of azobisisobutyronitrile and heated under nitrogen at 85°C for 3 ½ hours. The mixture is cooled and chromatographed on a single silica gel preparative layer chromatography plate eluting with chloroform. The remaining tributyltinhydride and tributyltinchloride move with the solvent front and the product is found at Rf of about 0.15 to 0.4. This band is collected and eluted from the silica gel with ethyl acetate. The mixture is chromatographed on a preparative layer silica gel chromatography plate eluting with chloroform affording 0.5 mg of 13-deoxy-22,23-dihydro-C-076-A1a-aglycone identified by mass spectrometry and nuclear magnetic resonance.

**Example 15**

**5-O-t-Butyldimethylsilyl-22,23-Dihydro-C-076-B1a-Aglycone**

50 mg of 22,23-dihydro-C-076-B1a-aglycone is dissolved in 1.1 ml of dimethylformamide containing 60 mg imidazole. While under nitrogen 75 mg of *t*-butyldimethylsilylchloride is added and the stoppered mixture is stirred overnight at room temperature. The reaction is quenched with 2 ml of water after dilution of the reaction mixture with 15 ml of ether. The aqueous phase is separated and extracted with 5 ml of ether. The combined organic phases are washed 5 times with 10 ml of water, the combined aqueous washes are extracted with 5 ml of ether, and the combined organic phases washed once again with 5 ml of water. The organic layer is dried over magnesium sulfate and evaporated to dryness *in vacuo* to an oil. The oil is chromatographed on two silica gel preparative layer chromatography plates eluting twice with methylene chloride. The slowest moving and most intense band is collected and washed from the silica gel with ethyl acetate. Lyophilization from benzene affords 36.3 mg of a white powder identified by nuclear magnetic resonance and mass spectrometry as 5-O-*t*-butyldimethylsilyl-22,23-dihydro-C-076-B1a-aglycone.

**Example 16**

**13-Chloro-13-Deoxy-5-O-t-Butyldimethylsilyl-22,23-Dihydro-C-076-B1a-Aglycone**

A solution of 35.5 mgf of 5-O-*t*-butyldimethylsilyl-22,23-dihydro-C-076-B1a-aglycone in 2.6 ml of methylene chloride containing 56 mg of 4-dimethylaminopyridine and 78 microliters (59 mg) of diisopropylethylamine is cooled to 0°C and treated with 75 mg of *o*-nitrobenzenesulfonyl chloride. The reaction mixture is stirred for 1 hour at 0°C, allowed to warm to room temperature and stirred for 3 hours. 3 ml of crushed ice is added to the reaction mixture followed by 4 ml of ether. The layers are separated and the aqueous phase washed with 4 ml of ether and the combined organic phases washed twice with 5 ml of water. The organic layer is dried over sodium sulfate and evaporated to dryness *in vacuo*. Benzene is added to the residue and azeotroped away. The product is isolated by preparative layer chromatography eluting with a 1 : 2 mixture of petroleum ether (b.p. 30 to 60°C) and chloroform to afford 5.4 mg of 13-chloro-13-deoxy-5-O-*t*-butyldimethylsilyl-22,23-dihydro-C-076-B1a-aglycone identified by mass spectrometry and nuclear magnetic resonance.

**Example 17**

**13-Deoxy-5-O-t-Butyldimethylsilyl-22,23-Dihydro-C76-B1a-Aglycone**

A solution of 13.2 mg of 13-chloro-13-deoxy-5-O-*t*-butyldimethylsilyl-22,23-dihydro-C-076-B 1 a-aglycone is combined with 0.7 ml of tributyltinhydride and 2.0 mg of azobisisobutyronitrile and heated to 85°C for 3 ½ hours under a blanket

of nitrogen. The reaction mixture is cooled and dissolved in 1.5 ml of methylene chloride and filtered through a column of silica gel eluting with methylene chloride. The tributyltinhydride and tributyltinchloride pass through the column upon washing with 250 ml of methylene chloride and the product remains on the column. The solvent is changed to ethyl acetate and the product eluted at the solvent front. The ethyl acetate solution is concentrated to an oil and the product purified by preparative layer chromatography on silica gel plates eluting with a 1 : 1 mixture of petroleum ether (b.p. 30 to 60°C) and methylene chloride to afford, after lyophilization from benzene, 8.2 mg of 13-deoxy-5-O-*t*-butyldimethylsilyl-22,23-dihydro-C-076-B1a-aglycone which is identified by mass spectrometry and nuclear magnetic resonance.

**Example 18**

**13-Deoxy-22,23-Dihydro-C-076-B1a-Aglycone**

A solution of 6.9 mg of 13-deoxy-5-O-*t*-butyldimethylsilyl-22,23-dihydro-C-076-B1a-aglycone in 0.6 ml of 1 % *p*-toluenesulfonic acid in methanol is stirred for 3 hours at room temperature. The reaction is quenched with 5 ml of ether and 1 ml of saturated aqueous potassium bicarbonate. The layers are separated and the aqueous phase washed with 2 ml of ether and the combined organic phases washed with water, dried over sodium sulfate and evaporated to dryness *in vacuo*. The oil is chromatographed on a single silica gel plate eluting with a 2 : 1 mixture of methylene chloride and petroleum ether (b.p. 30 to 60°C). After lyophilization there remains 4.5 mg of 13-deoxy-22,23-dihydro-C-076-B1a-aglycone identified by mass spectrometry and nuclear magnetic resonance.

**Example 19**

**13-Deoxy-23-O-t-Butyldimethylsilyl-C-076-A2a-Aglycone**

1 mg of 13-chloro-13-deoxy-23-O-*t*-butyldimethylsilyl-C-076-A2a-aglycone is dissolved in 50 microliters of toluene and 100 microliters of tributyltinhydride and 200 micrograms of azobisisobutyronitrile and heated at 60°C for 4 hours. The product is isolated by direct chromatography on a preparative layer silica gel chromatrography plate eluting with 1.5 % tetrahydrofuran in chloroform affording 13-deoxy-23-O-*t*-butyldimethylsilyl-C-076-A2a-aglycone which is identified by mass spectrometry.

**Example 20**

**5-O-Acetyl-13-Chloro-13-Deoxy-C-076-B1a-Aglycone**

25 mg of 13-chloro-13-deoxy-C-076-B1a-aglycone is dissolved in 0.6 ml of pyridine and 0.3 ml of acetic anhydride is added. The reaction is stirred at 20°C overnight. Ice is added to the reaction mixture, allowed to melt, and extracted with ether. The ether layer is washed with water, dried and concentrated *in vacuo*. The residue is purified by preparative layer chromatography on silica gel, eluting with chloroform, and the structure of 5-O-acetyl-13-chloro-13-deoxy-C-076-B1a-aglycone is confirmed by mass spectrometry and nuclear magnetic resonance.

**Example 21**

**5-O-Acetyl-13-Deoxy-C-076-B1a-Aglycone**

Following the procedure of Example 20 using 13-deoxy-C-076-B1a-aglycone in place of 13-chloro-13-deoxy-C-076-B1a-aglycone, there is obtained 5-O-acetyl-13-deoxy-C-076-B1a aglycone.

If propionic anhydride is employed in place of acetic anhydride in either of Examples 20 or 21, the analogous 5-O-propionyl compound is obtained.

**Example 22**

**5-O-t-Butyldimethylsilyl-13-Chloro-13-Deoxy-23-O-Acetyl-C-076-B2a-Aglycone**

A mixture of 20 mg of 5-O-*t*-butyldimethylsilyl-13-chloro-13-deoxy-C-076-B2a-aglycone, 0.8 ml of pyridine and 0.4 ml of acetic anhydride is heated in an oil bath for 2 hours at 100°C. The reaction mixture is cooled, ice is added, allowed to melt, and the precipitate collected by centrifugation. The solid material is dried, dissolved in methylene chloride and chromatographed on a preparative layer silica gel plate. The product is collected, dissolved in benzene and lyophilized affording 5-O-*t*-butyldimethylsilyl-13-chloro-13-deoxy-23-O-acetyl-C-076-B2a as a white fluffy solid.

**Example 23**

**5-O-t-Butyldimethylsilyl-13-Deoxy-23-O-Acetyl-C-076-B2a-Aglycone**

Following the procedure of Example 22 using 5-O-*t*-butyldimethylsilyl-13-deoxy-C-076-B2a-aglycone in place of 5-O-*t*-butyldimethylsilyl-13-chloro-13-deoxy-C-076-B2a-aglycone there is obtained 5-O-*t*-butyldimethylsilyl-13-deoxy-23-O-acetyl-C-076-B2a-aglycone.

**Example 24**

**13-Chloro-13-Deoxy-23-O-Acetyl-C-076-B2a-Aglycone**

10 mg of 5-O-*t*-butyldimethylsilyl-13-chloro-13-deoxy-23-O-acetyl-C-076-B2a-aglycone is dissolved in 0.5 ml of methanol containing 1 % by weight of *p*-toluenesulfonic acid dihydrate, and stirred at room temperature for 3 hours. To the reaction mixture is added 5 ml of ether and the solution washed with aqueous sodium bicarbonate solution, dried and concentrated under a stream of nitrogen to a colorless glass. The glass is further purified by preparative layer chromatography on silica gel eluting with chloroform, and affording pure 13-Chloro-13-deoxy-23-O-acetyl-C-076-B2a-aglycone.

**Example 25**

**13-Deoxy-23-O-Acetyl-C-076-B2a-Aglycone**

Following the procedure of Example 24 employing 5-O-*t*-butyldimethylsilyl-13-deoxy-23-O-acetyl-C-076-B2a-aglycone in place of 5-O-*t*-butyldimethylsilyl-13-chloro-13-deoxy-23-O-acetyl-C-076-B2a-aglycone, there is obtained 13-deoxy-23-O-acetyl-C-076-B2a-aglycone.

**Example 26**

**13-Chloro-13-Deoxy-5,23-Di-O-Acetyl-C-076-B2a-Aglycone**

50 mg of 13-chloro-13-deoxy-C-076-B2a-aglycone is dissolved in 1 ml of pyridine and 0.5 ml of acetic anhydride is added. The reaction mixture is heated for 2 hours at 100°C. Upon cooling to room temperature, ice water is added, producing a precipitate which is collected by filtration. The solid material is further purified by preparative layer chromatography eluting with 2 : 1 tetrahydrofuran in chloroform affording pure 13-chloro-13-deoxy-5,23-di-O-acetyl-C-076-B2a-aglycone.

**Example 27**

**13-Deoxy-5,23-Di-O-Acetyl-C-076-B2a-Aglycone**

Following the procedure of Example 26 using 13-deoxy-C-076-B2a-aglycone in place of 13-chloro-13-deoxy-C-076-B2a-aglycone, there is obtained 13-deoxy-5,23-di-O-acetyl-C-076-B2a-aglycone.

**Example 28**

**13-Deoxy-22,23-Dihydro-23-n-Butylthio-C-076-A1a-Aglycone**

A solution of 100 mg of 13-deoxy-C-076-A1-a-aglycone in a mixture of 9.4 ml of dioxane, 0.5 ml 30 of n-butanethiol and 0.1 ml of concentrated sulfuric acid is stirred at 18°C for 18 hours. The reaction mixture is diluted with 80 ml of ether washed with aqueous sodium bicarbonate solution, dried and concentrated *in vacuo* to a light glass. The glass is further purified on a preparative layer chromatography silica gel plate. The product is identified by mass spectrometry and nuclear magnetic resonance as 13-deoxy-22,23-dihydro-23-n-butylthio-C-076-A1a-aglycone.

**Example 29**

**13-Chloro-13-Deoxy-22,23-Dihydro-23-n-Butylthio-C-076-A1aAglycone**

Following the procedure of Example 28 employing 13-chloro-13-deoxy-C-076-A1a-aglycone in place of 13-deoxy-C-076-A1a-aglycone, one obtains 13-chloro-13-deoxy-22,23-dihydro-23-n-butylthio-C-076-A1a-aglycone.

**Example 30**

Following Example 28 employing equivalent amounts of methanethiol, isopropylthiol and *tert-* butylthiol, there is obtained 13-deoxy-22,23-dihydro-23-methylthio-C-076-A1a-aglycone, 13-deoxy-22,23-dihydro-23-isopropylthio-C-076-A1a-aglycone and 13-deoxy-22,23-dihydro-23-tert-butylthio-C-076-A1a-aglycone.

**Example 31**

**13-Deoxy-22,23-Dihydro-23-n-Butylsulfinyl-C-076-A1a-Aglycone**

A solution of 67 mg (0.1 mmoles) of 13-deoxy-22,23-dihydro-23-n-butylthio-C-076-A1a-aglycone in 1.0 ml of chloroform is stirred rapidly at 0°C. A second solution containing 19 mg (0.11 mmoles) of m-chloro perbenzoic acid in 0.5 ml of chloroform is added dropwise. The reaction mixture is allowed to reach 18°C, allowed to stand for 2 hours, then diluted with ether, washed with aqueous sodium bicarbonate solution, dried and concentrated under a stream of nitrogen to a colorless glass, which is identified as 13-deoxy-22,23-dihydro-23-n-butylsulfinyl-C-076-A1a-aglycone.

**Example 32**

**13-Deoxy-22,23-Dihydro-23-n-Butylsulfonyl-C-076-A1a-Aglycone**

Following the procedure of Example 31 employing twice the amount of *m*-chloro perbenzoic acid that is 38 mg (0.22 mmoles) in 1.0 ml of $HCCl_3$, affords 13-deoxy-22,23-dihydro-23-n-butylsulfonyl-C-076-A1a-aglycone.

**Example 33**

**13-Deoxy-22,23-Dihydro-23-Methoxy-C-076-B1a-Aglycone**

A solution of 100 mg of 13-deoxy-C76-B1a-aglycone in a mixture of 9.9 ml of methanol and 0.1 ml of concentrated sulfuric acid is maintained at 18°C for 20 hours. The reaction mixture is diluted with 100 ml of ether and washed with aqueous sodium bicarbonate solution. The solution is dried and concentrated *in vacuo* to a glass. The reaction product is further purified on a preparative layer silica gel chromatography plate and identified by nuclear magnetic resonance and mass spectrometry as 13-deoxy-22,23-dihydro-23-methoxy-C76-B1a-aglycone.

**Example 34**

Following the procedure of Example 33 but using ethanol, isopropanol or n-hexanol in place of methanol, 13-deoxy-22,23-dihydro-23-ethoxy-C-076-B1a-aglycone; 13-deoxy-22,23-dihydro-23-isopropoxy-C-076-B1a-aglycone, and 13-deoxy-22,23-dihydro-23-n-hexyloxy-C-076-B1a-aglycone are obtained.

**Example 35**

**13-Chloro-13-Deoxy-22,23-Dihydro-23-Methoxy-C-076-B1a-Aglycone.**

Following the procedure of Example 33 employing 13-chloro-13-deoxy-C-076-B1a-aglycone in place of 13-deoxy-C-076-B1a-aglycone there is obtained 13-chloro-deoxy-22,23-dihydro-23-methoxy-C-076-B1a-aglycone.

**Preparation 1**

**C-076 A1a-Aglycone**

100 mg of C-076 A1a is dissolved in 5 ml of dioxane, stirred and added at room temperature to a mixture of 0.1 ml of concentrated sulfuric acid, 1.9 ml of methanol and 3.0 ml of dioxane. The reaction mixture is stirred overnight at room temperature. 473 mg of solid sodium bicarbonate is added and the mixture stirred for 20 minutes. 3 ml of water is added and stirred for an additional 10 minutes. The reaction mixture is concentrated and 40 ml of chloroform is added and shaken. The aqueous layer is separated and extracted with 5 ml of chloroform. The organic layers are combined and washed once with dilute sodium chloride solution, dried over magnesium sulfate and evaporated to dryness *in vacuo.* Half of the residue is placed on 5 preparative layer chromatography silica gel plates and eluted with 2 % methanol in chloroform affording 4 bands of material. The remainder of the material was run on 2 preparative layer chromatography plates eluting with 2 % methanol in chloroform affording 4 bands similar to the first series. The second

fastest bands are removed from each of the plates, combined, extracted and evaporated to dryness *in vacuo*, and rechromatographed on a preparative layer chromatography silica gel plate eluting with 3 % tetrahydrofuran and chloroform affording 9.4 mg of a fluffy white solid which is identified by mass spectrometry as C-076 A1a-aglycone.

**Preparation 2**

**C-076-A2a-Aglycone**

2 g of C-076 A2a is combined with 40 ml of a 1 % (volume/volume) solution of concentrated sulfuric acid in methanol. The reaction mixture is stirred at room temperature for 17 hours and diluted with 300 ml of chloroform. The mixture is washed once with 30 ml of saturated sodium bicarbonate solution, once with 30 ml saturated sodium chloride solution, dried over magnesium sulfate and evaporated to dryness *in vacuo*. 5 ml of methanol is added to the residue and allowed to stand at room temperature overnight. Cooling of the mixture in ice causes the slow precipitation of crystals. A supernatant is removed and the solid crystals washed twice with 1 ml of cold methanol affording 340 mg of a white solid. The mother liquor and washings are evaporated down to a volume of about 2 ml and allowed to stand affording an additional crop of crystals. 630 mg of a white solid is obtained which is combined with the first batch of crystals and 8 ml of methanol and evaporated to a volume of 2.5 ml and allowed to stand for several hours. 910 mg of an off-white solid is obtained which mass spectrometry identifies as C-076 A2a-aglycone.

**Preparation 3**

**C-076-B2a Aglycone**

2 g of C-076-B2a is combined with 40 ml of a 1 % solution of concentrated sulfuric acid in methanol (volume/volume). The reaction mixture is stirred at room temperature for 17 hours. 300 ml of chloroform is added followed by 30 ml of an aqueous saturated sodium bicarbonate solution. The layers are separated and the organic layer washed with 30 ml of saturated sodium chloride solution, dried over magnesium sulfate and evaporated to dryness *in vacuo*. 5 ml of methanol is added to dissolve the residue and the mixture allowed to stand at room temperature and then cooled in an ice bath, whereupon crystallization occurred. The supernatant is removed and the residue washed twice with 1 ml portions of cold methanol and the solid crystals dried overnight and then *in vacuo* at 35°C affording 1.0 g of white crystals. A second crop is obtained by evaporating the mother liquors to a volume of 2 ml and allowing to stand overnight at room temperature. 2 ml of methanol is added and the mixture aged in an ice bath affording 140 mg of a yellow solid. The two solid fractions are combined and dissolved in boiling methanol, about 30 ml of methanol is required. The solution is filtered hot and concentrated to a volume of about 20 ml *in vacuo* whereupon solids begin to precipitate. The solution is filtered hot and the solid materials washed with methanol affording 340 mg of a white solid. The filtrates are boiled down to a volume of about 8 ml and set aside to crystallize at room temperature affording 433 mg of a white solid. Mass spectrometry shows the two fracions to be identical and to be identified as C-076-B2a-aglycone.

**Preparation 4**

**C-076-B1a-Aglycone**

100 mg of C-076 B1a is dissolved in 2.5 ml of dioxane and combined with 2.5 ml of a mixture prepared from 0.5 ml of water, 0.5 ml of concentrated sulfuric acid and 9.0 ml of dioxane. The reaction mixture is stirred at room temperature for 17 hours. 50 ml of ether and 25 ml of saturated aqueous sodium bicarbonate is added, the layers separated, and the organic layer washed with water and the water layer extracted with ether. The organic layers are combined, dried over sodium sulfate, and evaporated to dryness. Benzene is added and the solution again evaporated affording 60 mg of a yellow oil. The oil is chromatographed on a preparative layer chromatography silica gel plate, eluting with a 9 : 1 mixture of chloroform and tetrahydrofuran affording at an Rf of about 0.35, 16 mg of C-076 B1a-aglycone, which is identified by 300 MHz nuclear magnetic resonance.

**Preparation 5**

**22,23-Dihydro-C-076-A1a**

51.0 mg of C-076-A1 a and 14.4 mg of tris(triphenylphosphine)rhodium(I) chloride are combined in 3.5 ml of benzene and hydrogenated for 20 hours at room temperature under atmospheric pressure. The crude reaction mixture is chromatographed on a preparative layer chromatography plate eluting twice with 10 % tetrahydrofuran in chloroform. The product is removed from the support using ethyl acetate which is evaporated to dryness and the residue analyzed with 300 MHz nuclear magnetic resonance and mass spectroscopy indicating the preparation of 22, 23-dihydro-C-076 A1a.

**Preparation 6**

**22,23-Dihydro-C-076 B1a**

A solution of 1.007 g of C-076-B1a, 314 mg of tris(triphenylphosphine)rhodium(I)chloride and 33 ml of benzene is hydrogenated for 21 hours at room temperature under atmospheric hydrogen pressure. The solvent is removed *in vacuo* and the residue dissolved in a 1 : 1 mixture of methylene chloride and ethyl acetate and filtered. The filtrate is placed on a column of 60 g of silica gel eluting with a 1 : 1 mixture of methylene chloride and ethyl acetate taking 10 ml fractions. Fractions 14 - 65 are combined and evaporated to dryness affording 1.118 g of a solid material which is indicated by high pressure liquid chromatography to be a 60/40 mixture of the hydrogenated product and starting material. The mixture is rehydrogenated in 55 ml of benzene adding 310 mg of tris(triphenylphosphine) rhodium(I)chloride and stirring for 21 hours at room temperature under atmospheric hydrogen pressure. The solvent is removed *in vacuo* and the residue chromatographed on 80 g of silica gel using 40 : 60 mixture of ethyl acetate and methylene chloride as eluant. 10 ml fractions are taken and the product appears in fractions 26 - 80. These fractions are combined and evaporated to dryness *in vacuo* affording a yellow oil. The oil is dissolved in benzene and lyophilized affording a pale yellow powder which is identified as 22,23-dihydro-C-076-B1a by mass spectrometry and 300 MHz nuclear magnetic resonance. 0.976 g of product is obtained.

**Preparation 7**

**22,23-Dihydro-C-076-A1a Aglycone**

10.1 mg of 22,23-dihydro-C-076 A1 a is stirred for 20 hours in 1.1 ml of 1 % sulfuric acid in methanol at room temperature. The reaction mixture is treated as in Preparation 6 affording an oil which is purified by preparative layer chromatography on silica gel eluting with 5 % tetrahydrofuran in chloroform. The product is removed from the chromatography plate and lyophilized from benzene affording 4.2 mg of a white powder which 300 MHz nuclear magnetic resonance and mass spectrometry indicate to be 22,23-dihydro-C-706-A1a aglycone.

**Preparation 8**

**22,23-Dihydro-C-076-B1a-Aglycone**

0.486 g of 22,23-dihydro-C-076-B1a is added to a stirred solution of 50 ml of 1 % sulfuric acid in methanol and the reaction mixture stirred for 13 hours at room temperature. The reaction mixture is diluted with 250 ml of methylene chloride and washed with 50 ml of saturated aqueous potassium bicarbonate and 50 ml of water. The aqueous layer is washed twice with 20 ml portions of methylene chloride and the combined organic phases are dried with saturated brine and sodium sulfate and evaporated to dryness *in vacuo* affording 0.480 g of a pale yellow foam. The foam is dissolved in 4 ml of methylene chloride and placed on 4 preparative layer chromatography silica gel plates and eluted 4 times with 4 % tetrahydrofuran and chloroform. The product is recovered from the silica gel plates affording an oily residue which is lyophilized from benzene affording 255.8 mg of a white solid. Traces of methyl oleandroside are indicated to be present in the solid material. The white solid is then lyophilized again from benzene and placed under high vacuum for 20 hours to remove the impurity affording 22,23-dihydro-C-076-B1 a-aglycone.

Based on taxonomic studies, the microorganism capable of producing C-076 compounds are of a new species of the genus Streptomyces, which has been named *Streptomyces avermitilis*. One such culture, isolated from soil, is designated MA-4680 in the culture collection of Merck & Co. Inc.. Rahway, New Jersey. A C-076-producing sample of this culture has been deposited in the permanent culture collection of the Fermentation Section of the Northern Utilization Research Branch, U.S. Department of Agriculture at Peoria, Illinois, and has been assigned the accession number NRRL 8 165. A sample of NRRL 8 165 has also been deposited, without restriction as to availability, in the permanent culture collection of the American Type Culture Collection at 12 301 Parklawn Drive, Rockville, Maryland 20 852, and has been assigned the accession number ATCC 31,267.

The morphological and cultural characteristics of *Streptomyces avermitilis* are set forth below:

Morphology; Sporophores form spirals as side branches on aerial mycelia. Spirals are compact but become more open as culture ages. Spores are in chains or more than 15 spores and are usually spherical to oval at 970 X magnification. Sporulation is observed on oatmeal agar, glycerol-asparagine agar, salts-starch agar and egg albumin agar. Spore surface is smooth as seen by electron microscopy.

Oatmeal agar

| | |
|---|---|
| Vegetative growth: | Reverse - very dark brown |
| Aerial mycelium: | Powdery, brownish gray (4li)* mixed with white. |
| Soluble pigment: | Brown |

Czapek Dox agar (sucrose nitrate agar)

| | |
|---|---|
| Vegetative growth: | Poor, colorless |
| Aerial mycelium: | Scant, grayish |
| Soluble pigment: | Light grayish tan |
| **Egg albumin agar** | |
| Vegetative growth: | Tan |
| Aerial mycelium: | Moderate, light grayish-yellow brown (3ge)* mixed with white. |
| Soluble pigment: | Light yellowish tan |
| **Glycerol asparagine agar** | |
| Vegetative growth: | Reverse - yellowish brown |
| Aerial mycelium: | Powdery, brownish gray (4li)* mixed with white. |
| Soluble pigment | Light, yellowish brown |
| **Inorganic salts-starch agar** | |
| Vegetative growth: | Reverse - grayish yellowish brown. |
| Aerial mycelium: | Powdery, light brownish gray (4ig)* edged with darker brownish gray(4li)* |
| Soluble pigment: | Light yellowish brown |
| **Yeast extract-dextrose + salts agar** | |
| Vegetative growth: | Reverse - dark brown |
| Aerial mycelium: | Moderate, brownish white |
| Soluble pigment: | Brown |
| **Yeast extract-malt extract agar** | |
| Vegetative growth: | Reverse - dark brown |
| Aerial mycelium: | Moderate brownish white |
| Soluble pigment | Brown |
| **Peptone-iron-yeast extract agar** | |
| Vegetative growth: | Dark brown |
| Aerial Mycelium: | None |
| Soluble pigment: | Dark brown to black |
| Melanin: | Positive |
| H S production | Positive |
| **Nutrient agar** | |
| Vegetative growth: | Tan |
| Aerial mycelium: | Sparse grayish |
| Soluble pigment: | Light brown |
| **Nutrient starch agar** | |
| Vegetative growth: | Tan |
| Aerial mycelium: | Sparse, grayish white |
| Soluble pigment: | Light brown |
| Hydrolysis of starch: | Good |
| **Potato plug** | |
| Vegetative growth: | Tan |
| Aerial mycelium: | Brown mixed with grayish white |
| Soluble pigment: | Grayish brown |
| **Loeffler's Blood serum** | |
| Vegetative growth: | Grayish tan |
| Aerial mycelium: | None |
| Soluble pigment: | Some browning of medium |
| Liquefaction: | None |
| **Nutrient tyrosine agar** | |
| Vegetative growth: | Reverse - dark brown to black |
| Aerial mycelium: | Sparse, grayish |
| Soluble pigment: | Dark brown |
| Decomposition of tyrosine: | None |

Carbon utilization
Pridham-Gottlieb basal medium + 1 % carbon source;
+ = growth; no growth as compared to negative control (no carbon source).

| | |
|---|---|
| Glucose | + |
| Arabinose | + |
| Cellulose | - |
| Fructose | + |
| Inositol | + |
| Lactose | + |
| Maltose | + |
| Mannitol | + |
| Mannose | + |
| Raffinose | + |
| Rhamnose | + |
| Sucrose | + |
| Xylose | + |

| | |
|---|---|
| **Nutrient gelatin agar** | |
| Vegetative growth: | Tan |
| Aerial mycelium: | Sparse, grayish white |
| Soluble pigment: | Light brown |
| Liquefaction of gelatin: | Good |
| **Gelatin stabs** | |
| Vegetative growth: | Brown ring |
| Aerial mycelium: | None |
| Soluble pigment: | Greenish brown |
| Liquefaction of gelatin: | Complete |
| **Skim milk agar** | |
| Vegetative growth: | Dark brown |
| Aerial mycelium: | None |
| Soluble pigment: | Dark brown |
| Hydrolysis of casein: | Good |
| **Litmus milk** | |
| Vegetative growth: | Dark brown growth ring |
| Aerial mycelium: | None |
| Color: | Dark brown |
| Coagulation and/or peptonization: | Complete peptonization; becoming alkaline (pH 8.1) |
| **Skim milk** | |
| Vegetative growth: | Dark brown growth ring |
| Aerial mycelium: | None |
| Soluble pigment: | Dark brown |
| Coagulation and/or peptonization: | Complete peptonization; becoming alkaline (pH 8.0). |

Temperature range: (Yeast extract-dextrose + salts agar)

280C - Good vegetative growth and aerial mycelia
37°C - Good vegetative growth and aerial mycelia
50°C - No growth

Oxygen requirement: (Stab culture in yeast extract-dextrose + salts agar)
Aerobic

All readings taken after three weeks at 28°C unless noted otherwise. pH of all media approximately neutral (6.8 - 7.2) Color number designations (*) taken from Color Harmony Manual, 1958, 4th Edition Container Corporation of America, Chicago, Illinois.

A careful comparison of the foregoing data with published descriptions including Bergey's Manual of Determinative Bacteriology (Eighth Edition) of known microorganisms reveals significant differences that indicate that the microorganism should be classified as a new species. On this basis, it was designed *Streptomyces avermitilis*.

Other organisms can also be used to produce C-076, e.g. mutants obtained by mutating agents such as X-ray irradiation, ultraviolet irradiation or nitrogen mustards.

A culture of one such organism was isolated after irradiating *S. avermitilis* with ultraviolet light. A lyophilized tube and a frozen vial of this culture have been deposited in the permanent Culture Collection of the American Type Culture Collection, and they have been assigned the accession numbers 31 272 and 31 271 respectively. Slightly higher fermentation yields of C-076 have been obtained using this frozen stock as inoculum.

## Claims

1. A compound obtainable by the reaction of one of C-076 A1a, C-076 A2a, C-076 B1a, C-076 B2a, C-076 A1b, C-076 A2b, C-076 B1b and C-076 B2b, as follows:

(a) removing the α-*L*-oleandrosyl-α-*L*-oleandrose group by hydrolysis;
(b) replacing the 13-hydroxy group resulting from step (a) with a 13-halo group by reaction with a sufficiently reactive benzenesulfonyl halide in the presence of a base;
(c) optionally removing the 13-halo group with a selective reducing agent;
(d) optionally reducing the 22,23-double bond on the A1a, B1a, A1b and B1b compounds to a single bond by hydrogenation using a solvent and a catalyst of the formula $[(R_5)_3P]_3RhX$ where $R_5$ is loweralkyl, phenyl, or loweralkyl-substituted phenyl and X is a halogen;
(e) optionally $C_{2-6}$ alkanoylating the 5- or 23- hydroxy group of the A2a, B1a, A2b or B1b compound or on one or both of the 5- and 23- hydroxy groups in the B2a or B2b compound;
(f) optionally preparing a 23-($C_{1-6}$ alkoxy) or 23-($C_{16}$ alkylthio) derivative of the A1a, B1a, A1b or B1b type compounds by reaction with a $C_{1-6}$ alkanol or $C_{1-6}$ alkylthiol in the presence of an acid; and
(g) optionally oxidizing a 23-($C_{1-6}$ alkylthio) derivative prepared in step (f) to a $C_{1-6}$ alkylsulphinyl or a $C_{1-6}$ alkylsulphonyl group.

2. A compound obtainable by the reaction of C-076 A1a as follows:

(a) removing the α-*L*-oleandrosyl-α-*L*-oleandrose group by hydrolysis;
(b) replacing the 13-hydroxy group resulting from step (a) with a 13-halo group by reaction with a sufficiently reactive benzenesulfonyl halide in the presence of a base;
(c) optionally removing the 13-halo group with a selective reducing agent;
(d) optionally reducing the 22,23-double bond to a single bond by hydrogenation using a solvent and a catalyst of the formula set forth in step (d) of Claim 1;
(e) optionally preparing a 23-($C_{1-6}$ alkoxy) or 23-($C_{1-6}$ alkylthio) derivative by reaction with a $C_{1-6}$ alkanol or $C_{1-6}$ alkylthiol in the presence of an acid; and
(f) optionally oxidizing a 23-($C_{1-6}$ alkylthio) derivative prepared in step (e) to a $C_{1-6}$ alkylsulphinyl or a $C_{1-6}$ alkylsulphonyl group.

3. A compound obtainable by the reaction of C-076 A1b as follows:

(a) removing the α-*L*-oleandrosyl-α-*L*-oleandrose group by hydrolysis;
(b) replacing the 13-hydroxy group resulting from step (a) with a 13-halo group by reaction with a sufficiently reactive benzenesulfonyl halide in the presence of a base;
(c) optionally removing the 13-halo group with a selective reducing agent;
(d) optionally reducing the 22,23-double bond to a single bond by hydrogenation using a solvent and a catalyst of the formula set forth in step (d) of Claim 1;
(e) optionally preparing a 23-($C_{1-6}$ alkoxy) or 23-($C_{1-6}$ alkylthio) derivative by reaction with a $C_{1-6}$ alkanol or $C_{1-6}$ alkylthiol in the presence of an acid; and
(f) optionally oxidizing a 23-($C_{1-6}$ alkylthio) derivative prepared in step (e) to a $C_{1-6}$ alkylsulphinyl or a $C_{1-6}$ alkylsulphonyl group.

4. A compound obtainable by the reaction of C-076 A2a as follows:

(a) removing the α-*L*-oleandrosyl-α-*L*-oleandrose group by hydrolysis;
(b) replacing the 13-hydroxy group resulting from step (a) with a 13-halo group by reaction with a sufficiently reactive benzenesulfonyl halide in the presence of a base;
(c) optionally removing the 13-halo group with a selective reducing agent; and
(d) optionally $C_{2-6}$ alkanoylating the 23-hydroxy group.

5. A compound obtainable by the reaction of C-076 A2b as follows:

(a) removing the α-*L*-oleandrosyl-α-*L*-oleandrose group by hydrolysis;
(b) replacing the 13-hydroxy group resulting from step (a) with a 13-halo group by reaction with a sufficiently reactive

benzenesulfonyl halide in the presence of a base;
(c) optionally removing the 13-halo group with a selective reducing agent; and
(d) optionally $C_{2-6}$ alkanoylating the 23-hydroxy group.

6. A compound obtainable by the reaction of C-076 B1a as follows:

(a) removing the α-*L*-oleandrosyl-α-*L*-oleandrose group by hydrolysis;
(b) replacing the 13-hydroxy group resulting from step (a) with a 13-halo group by reaction with a sufficiently reactive benzenesulfonyl halide in the presence of a base;
(c) optionally removing the 13-halo group with a selective reducing agent;
(d) optionally reducing the 22,23-double bond to a single bond by hydrogenation using a solvent and a catalyst of the formula set forth in step (d) of Claim 1;
(e) optionally $C_{2-6}$ alkanoylating the 5-hydroxy group;
(f) optionally preparing a 23-($C_{1-6}$ alkoxy) or 23-($C_{1-6}$ alkylthio) derivative by reaction with a $C_{1-6}$ alkanol or $C_{1-6}$ alkylthiol in the presence of an acid; and
(g) optionally oxidizing a 23-($C_{1-6}$ alkylthio) derivative prepared in step (f) to a $C_{1-6}$ alkylsulphinyl or a $C_{1-6}$ alkylsulphonyl group.

7. A compound obtainable by the reaction of C-076 B1b as follows:

(a) removing the α-*L*-oleandrosyl-α-*L*-oleandrose group by hydrolysis;
(b) replacing the 13-hydroxy group resulting from step (a) with a 13-halo group by reaction with a sufficiently reactive benzenesulfonyl halide in the presence of a base;
(c) optionally removing the 13-halo group with a selective reducing agent;
(d) optionally reducing the 22,23-double bond to a single bond by hydrogenation using a solvent and a catalyst of the formula set forth in step (d) of Claim 1;
(e) optionally $C_{2-6}$ alkanoylating the 5-hydroxy group;
(f) optionally preparing a 23-($C_{1-6}$ alkoxy) or 23-($C_{1-6}$ alkylthio) derivative by reaction with a $C_{1-6}$ alkanol or $C_{1-6}$ alkylthiol in the presence of an acid; and
(g) optionally oxidizing a 23-($C_{1-6}$ alkylthio) derivative prepared in step (f) to a $C_{1-6}$ alkylsulphinyl or a $C_{1-6}$ alkylsulphonyl group.

8. A compound obtainable by the reaction of C-076 B2a as follows:

(a) removing the α-*L*-oleandrosyl-α-*L*-oleandrose group by hydrolysis;
(b) replacing the 13-hydroxy group resulting from step (a) with a 13-halo group by reaction with a sufficiently reactive benzenesulfonyl halide in the presence of a base;
(c) optionally removing the 13-halo group with a selective reducing agent; and
(d) optionally $C_{2-6}$ alkanoylating one or both of the 5- and 23-hydroxy groups.

9. A compound obtainable by the reaction of C-076 B2b as follows:

(a) removing the α-*L*-oleandrosyl-α-*L*-oleandrose group by hydrolysis;
(b) replacing the 13-hydroxy group resulting from step (a) with a 13-halo group by reaction with a sufficiently reactive benzenesulfonyl halide in the presence of a base;
(c) optionally removing the 13-halo group with a selective reducing agent; and
(d) optionally $C_{2-6}$ alkanoylating one or both of the 5- and 23-hydroxy groups.

10. A compound obtainable by the reaction of C-076 B1b as follows:

(a) removing the α-*L*-oleandrosyl-α-*L*-oleandrose group by hydrolysis;
(b) replacing the 13-hydroxy group resulting from step (a) with a 13-halo group by reaction with a sufficiently reactive benzenesulfonyl halide in the presence of a base;
(c) removing the 13-halo group with a selective reducing agent; and
(d) reducing the 22,23-double bond to a single bond by hydrogenation using a solvent and a catalyst of the formula set forth in step (d) of Claim 1.

11. A compound as claimed in Claim 1 for use in the treatment of parasitic infections.

12. A method of preparing a compound as claimed in Claim 1 wherein step (c) is carried out by treating the 13-halo compound with a trialkyltin hydride in the presence of a free-radical initiator.

**Revendications**

1. Composé que l'on peut obtenir par la réaction d'un des composés C-076 A1a, C-076 A2a, C-076 B1a, C-076 B2a, C-076 A1b, C-076 A2b, C-076 B1b et C-076 B2b, comme suit:

(a) on retire le groupe α-*L*-oléandrosyl-α-*L*-oléandrose par hydrolyse;
(b) on remplace le groupe 13-hydroxy résultant de l'étape (a) par un groupe 13-halo par réaction avec un halogénure de benzènesulfonyle suffisamment réactif en présence d'une base;
(c) si on le désire on retire le groupe 13-halo avec un agent réducteur sélectif;
(d) si on le désire on réduit la double liaison 22,23 sur les composés A1a, B1a, A1b et B1b en une liaison simple par hydrogénation, en utilisant un solvant et un catalyseur de formule $[(R_5)_3P]_3RhX$ où $R_5$ est un alcoyle inférieur, un phényle ou un phényle substitué par un alcoyle inférieur et X est un halogène;
(e) si on le désire on traite avec un alcanoyle en $C_2$ à $C_6$ le groupe 5- ou 23-hydroxy du composé A2a, B1a, A2b ou B1b ou l'un des groupes hydroxy en 5 et en 23, ou les deux, dans le composé B2a ou B2b;
(f) si on le désire on prépare un dérivé 23-(alcoxy en $C_1$ à $C_6$) ou 23-(alcoylthio en $C_1$ à $C_6$) des composés du type A1a, B1a, A1b ou B1b par réaction avec un alcanol en $C_1$ à $C_6$ ou un alcoylthiol en $C_1$ à $C_6$ en présence d'un acide; et
(g) si on le désire on oxyde un dérivé 23-(alcoylthio en $C_1$ à $C_6$) préparé dans l'étape (f) en un groupe alcoyle en $C_1$ à $C_6$-sulfinyle ou alcoyle en $C_1$ à $C_6$-sulfonyle.

2. Composé que l'on peut obtenir par la réaction de C-076 A1a comme suit:

(a) on retire le groupe α-*L*-oléandrosyl-α-*L*-oléandrose par hydrolyse;
(b) on remplace le groupe 13-hydroxy résultant de l'étape (a) par un groupe 3-halo par réaction avec un halogénure de benzénesulfonyle suffisamment réactif en présence d'une base;
(c) si on le désire on retire le groupe 13-halo avec un agent réducteur sélectif;
(d) si on le désire on réduit la double liaison 22,23 en une liaison simple par hydrogénation en utilisant un solvant et un catalyseur de la formule présentée dans l'étape (d) de la revendication 1;
(e) si on le désire on prépare un dérivé 23-(alcoxy en $C_1$ à $C_6$) ou 23-(alcoylthio en $C_1$ à $C_6$) par réaction avec un alcanol en $C_1$ à $C_6$ ou un alcoylthiol en $C_1$ à $C_6$ en présence d'un acide; et
(f) si on le désire on oxyde un dérivé 23-(alcoylthio en $C_1$ à $C_6$ préparé dans l'étape (e) en un groupe alcoyle en $C_1$ à $C_6$-sulfinyle ou un groupe alcoyle en $C_1$ à $C_6$-sulfonyle.

3. Composé que l'on peut obtenir par la réaction de C-076 A1b comme suit:

(a) on retire le groupe α-*L*-oléandrosyl-α-*L*-oléandrose par hydrolyse;
(b) on remplace le groupe 13-hydroxy résultant de l'étape (a) par un groupe 13-halo par réaction avec un halogénure de benzénesulfonyle suffisamment réactif en présence d'une base;
(c) si on le désire on retire le groupe 13-halo avec un agent réducteur sélectif;
(d) si on le désire on réduit la double liaison 22,23 en une liaison simple par hydrogénation en utilisant un solvant et un catalyseur de la formule présentée dans l'étape (d) de la revendication 1;
(e) si on le désire on prépare un dérivé 23-(alcoxy en $C_1$ à $C_6$) ou 23-(alcoylthio en $C_1$ à $C_6$) par réaction avec un alcanol en $C_1$ à $C_6$ ou un alcoylthiol en $C_1$ à $C_6$ en présence d'un acide; et
(f) si on le désire on oxyde un dérivé 23-(alcoylthio en $C_1$ à $C_6$) préparé dans l'étape (e) en un groupe alcoyle en $C_1$ à $C_6$-sulfinyle ou alcoyle en $C_1$ à $C_6$-sulfonyle.

4. Composé que l'on peut obtenir par réaction de C-076 A2a comme suit:

(a) on retire le groupe α-*L*-oléandrosyl-α-*L*-oléandrose par hydrolyse;
(b) on remplace le groupe 13-hydroxy résultant de l'étape (a) par un groupe 13-halo par réaction avec un halogénure de benzènesulfonyle suffisamment réactif en présence d'une base;
(c) si on le désire on retire le groupe 13-halo avec un agent réducteur sélectif; et
(d) si on le désire on traite avec un alcanoyle en $C_2$ à $C_6$ le groupe 23-hydroxy.

5. Composé que l'on peut obtenir par la réaction de C-076 A2b comme suit:

(a) on retire le groupe α-*L*-oléandrosyl-α-*L*-oléandrose par hydrolyse;
(b) on remplace le groupe 13-hydroxy résultant de l'étape (a) par un groupe 13-halo par réaction avec un halogénure de benzènesulfonyle suffisamment réactif en présence d'une base;
(c) si on le désire on retire le groupe 13-halo avec un agent réducteur sélectif; et
(d) si on le désire on traite avec un alcanoyle en $C_2$ à $C_6$ le groupe 23-hydroxy.

6. Composé que l'on peut obtenir par la réaction de C-076 B1a comme suit:

(a) on retire le groupe α-*L*-oléandrosyl-α-*L*-oléandrose par hydrolyse;

(b) on remplace le groupe 13-hydroxy résultant de l'étape (a) par un groupe 13-halo par réaction avec un halogénure de benzènesulfonyle suffisamment réactif en présence d'une base;
(c) si on le désire on retire le groupe 13-halo avec un agent réducteur sélectif;
(d) si on le désire on réduit la double liaison 22,23 en une liaison simple par hydrogénation en utilisant un solvant et un catalyseur de la formule présentée dans l'étape (d) de la revendication 1;
(e) si on le désire on traite avec un alcanoyle en $C_2$ à $C_6$ le groupe 5-hydroxy;
(f) si on le désire on prépare un dérivé 23-(alcoxy en $C_1$ à $C_6$) ou 23-(alcoylthio en $C_1$ à $C_6$) par réaction avec un alcanol en $C_1$ à $C_6$ ou un alcoylthiol en $C_1$ à $C_6$ en présence d'un acide; et
(g) si on le désire onoxyde un dérivé 23-(alcoylthio en $C_1$ à $C_6$) préparé dans l'étape (f) en un groupe alcoyle en $C_1$ à $C_6$-sulfinyle ou alcoyle en $C_1$ à $C_6$-sulfonyle.

7. Composé que l'on peut obtenir par la réaction de C-076 B1b comme suit:

(a) on retire le groupe α-*L*-oléandrosyl-α-*L*-oléandrose par hydrolyse;
(b) on remplace le groupe 13-hydroxy résultant de l'étape (a) par un groupe 13-halo par réaction avec un halogénure de benzènesulfonyle suffisamment réactif en présence d'une base;
(c) si on le désire on retire le groupe 13-halo avec un agent réducteur sélectif;
(d) si on le désire on réduit la double liaison 22,23 en une liaison simple par hydrogénation en utilisant un solvant et un catalyseur de la formule présentée dans l'étape (d) de la revendication 1;
(e) si on le désire on traite avec un alcanoyle en $C_2$ à $C_6$ le groupe 5-hydroxy;
(f) si on le désire on prépare un dérivé 23-(alcoxy en $C_1$ à $C_6$) ou 23-(alcoylthio en $C_1$ à $C_6$) par réaction avec un alcanol en $C_1$ à $C_6$ ou un alcoylthiol en $C_1$ à $C_6$ en présence d'un acide; et
(g) si on le désire on oxyde un dérivé 23-(alcoylthio en $C_1$ à $C_6$) préparé dans l'étape (f) en un groupe alcoyle en $C_1$ à $C_6$-sulfinyle ou alcoyle en $C_1$ à $C_8$-sulfonyle.

8. Composé que l'on peut obtenir par la réaction de C-076 B2a comme suit:

(a) on retire le groupe α-*L*-oléandrosyl-α-*L*-oléandrose par hydrolyse;
(b) on remplace le groupe 13-hydroxy résultant de l'étape (a) par un groupe 13-halo par réaction avec un halogénure de benzènesulfonyle suffisamment réactif en présence d'une base;
(c) si on le désire on retire le groupe 13-halo avec un agent réducteur sélectif; et
(d) si on le désire on traite avec un alcanoyle en $C_2$ à $C_6$ un des groupes 5- et 23-hydroxy ou les deux.

9. Composé que l'on peut obtenir par la réaction de C-076 B2b comme suit:

(a) on retire le groupe α-*L*-oléandrosyl-α-*L*-oléandrose par hydrolyse;
(b) on remplace le groupe 13-hydroxy résultant de l'étape (a) par un groupe 13-halo par réaction avec un halogénure de benzènesulfonyle suffisamment réactif en présence d'une base;
(c) si on le désire on retire le groupe 13-halo avec un agent réducteur sélectif; et
(d) si on le désire on traite avec un alcanoyle en $C_2$ à $C_6$ un des groupes 5- et 23-hydroxy ou les deux.

10. Composé que l'on peut obtenir par la réaction de C-076 B1b comme suit:

(a) on retire le groupe α-*L*-oléandrosyl-α-*L*-oléandrose par hydrolyse;
(b) on remplace le groupe 13-hydroxy résultant de l'étape (a) par un groupe 13-halo par réaction avec un halogénure de benzènesulfonyle suffisamment réactif en présence d'une base;
(c) on retire le groupe 13-halo avec un agent réducteur sélectif; et
(d) on réduit la double liaison 22,23 en une liaison simple par hydrogénation en utilisant un solvant et un catalyseur de la formule présentée dans l'étape (d) de la revendication 1.

11. Composé selon la revendication 1 aux fins d'application dans le traitement des infections parasitaires.

12. Procédé de préparation d'un composé selon la revendication 1 où l'étape (c) est conduite par le composé 13-halo avec un hydrure de trialcoyl-étain en présence d'un initiateur de radicaux libres.

**Patentansprüche**

1. Verbindung, erhältlich durch Umsetzung von einer der Verbindungen C-076 A1a, C-076 A2a, C-076 B1a, C-076 B2a, C-076 A1b, C-076 A2b, C-076 B1b und C-076 B2b, auf folgende Weise:

(a) Entfernen der α-*L*-Oleandrosyl-α-*L*-oleandrosegruppe durch Hydrolyse;
(b) Ersetzen der in Stufe (a) erhaltenen 13-Hydroxygruppe durch eine 13-Halogengruppe durch Umsetzung mit einem ausreichend reaktiven Benzolsulfonylhalogenid in Gegenwart einer Base;
(c) gegebenenfalls Entfernen der 13-Halogengruppe mit einem selektiven Reduktionsmittel;

(d) gegebenenfalls Reduzieren der 22,23-Doppelbindung an den A1a-, B1a-, A1b-und B1b-Verbindungen zu einer Einfachbindung durch Hydrieren unter Verwendung eines Lösungsmittels und eines Katalysators der Formel $[(R_5)_3P]_3RhX$, wobei $R_5$ nieder-Alkyl, Phenyl oder durch nieder-Alkyl substituiertes Phenyl und X Halogen bedeutet;
(e) gegebenenfalls $C_{2-6}$-Alkanoylieren der 5- oder 23-Hydroxygruppe der A2a- B1a-, A2b- oder B1b-Verbindung oder einer oder beider der 5- und 23-Hydroxygruppen in der B2a- oder B2b-Verbindung;
(f) gegebenenfalls Herstellen eines 23-($C_{1-6}$-Alkoxy)- oder 23-($C_{1-6}$-Alkylthio)-derivats der Verbindungen vom A1a-, B1a-, A1b- oder B1b-Typ durch Umsetzung mit einem $C_{1-6}$-Alkanol oder $C_{1-6}$-Alkylthiol in Gegenwart einer Säure;
(g) gegebenenfalls Oxidieren eines in Stufe (f) hergestellten 23-($C_{1-6}$-Alkylthio)-derivats zu einer $C_{1-6}$-Alkylsulfinyl- oder einer $C_{1-6}$-Alkylsulfonyl gruppe.

2. Verbindung, erhältlich durch Umsetzung von C-076 A1a auf folgende Weise:

(a) Entfernen der α-*L*-Oleandrosyl-α-*L*-oleandrosegruppe durch Hydrolyse;
(b) Ersetzen der in Stufe (a) erhaltenen 13-Hydroxygruppe durch eine 13-Halogengruppe durch Umsetzung mit einem ausreichend reaktiven Benzolsulfonylhalogenid in Gegenwart einer Base;
(c) gegebenenfalls Entfernen der 13-Halogengruppe mit einem selektiven Reduktionsmittel;
(d) gegebenenfalls Reduzieren der 22,23-Doppelbindung zu einer Einfachbindung durch Hydrieren unter Verwendung eines Lösungsmittels und eines Katalysators der in Stufe (d) von Anspruch 1 angegebenen Formel;
(e) gegebenenfalls Herstellen eines 23-($C_{1-6}$-Alkoxy)-oder 23-($C_{1-6}$-Alkylthio)-derivats durch Umsetzung mit einem $C_{1-6}$-Alkanol oder $C_{1-6}$-Alkylthiol in Gegenwart einer Säure und
(f) gegebenenfalls Oxidieren eines in Stufe (e) erhaltenen 23-($C_{1-6}$-Alkylthio)-derivats in eine $C_{1-6}$-Alkylsulfinyl- oder $C_{1-6}$-Alkylsulfonylgruppe.

3. Verbindung, erhältlich durch Umsetzung von C-076 A1b auf folgende Weise:

(a) Entfernen der α-*L*-Oleandrosyl-α-*L*-oleandrosegruppe durch Hydrolyse;
(b) Ersetzen der in Stufe (a) erhaltenen 13-Hydroxygruppe durch eine 13-Halogengruppe durch Umsetzung mit einem ausreichend reaktiven Benzolsulfonylhalogenid in Gegenwart einer Base;
(c) gegebenefalls Entfernen der 13-Halogengruppe mit einem selektiven Reduktionsmittel;
(d) gegebenenfalls Reduzieren der 22,23-Doppelbindung zu einer Einfachbindung durch Hydrieren unter Verwendung eines Lösungsmittels und eines Katalysators der in Stufe (d) von Anspruch 1 angegebenen Formel;
(e) gegebenenfalls Herstellen eines 23-($C_{1-6}$-Alkoxy)- oder 23-($C_{1-6}$-Alkylthio)-derivats durch Umsetzung mit einem $C_{1-6}$-Alkanol oder $C_{1-6}$-Alkylthiol in Gegenwart einer Säure; und
(f) gegebenenfalls Oxidieren eines in Stufe (e) hergestellten 23-($C_{1-6}$-Alkylthio)-derivats zu einer $C_{1-6}$-Alkylsulfinyl- oder $C_{1-6}$-Alkylsulfonylgruppe.

4. Verbindung, erhältlich durch Umsetzung von C-076 A2a auf folgende Weise:

(a) Entfernen der α-*L*-Oleandrosyl-α-*L*-oleandrosegruppe durch Hydrolyse;
(b) Ersetzen der in Stufe (a) erhaltenen 13-Hydroxygruppe durch eine 13-Halogengruppe durch Umsetzung mit einem ausreichend reaktiven Benzolsulfonylhalogenid in Gegenwart einer Base;
(c) gegebenenfalls Entfernen der 13-Halogengruppe mit einem selektiven Reduktionsmittel; und
(d) gegebenenfalls $C_{2-6}$-Alkanoylieren der 23-Hydroxygruppe.

5. Verbindung, erhältlich durch Umsetzung von C-076 A2b auf folgende Weise:

(a) Entfernen der α-*L*-Oleandrosyl-α-oleandrosegruppe durch Hydrolyse;
(b) Ersetzen der in Stufe (a) erhaltenen 13-Hydroxygruppe durch eine 13-Halogengruppe durch Umsetzung mit einem ausreichend reaktiven Benzolsulfonylhalogenid in Gegenwart einer Base;
(c) gegebenenfalls Entfernen der 13-Halogengruppe mit einem selektiven Reduktionsmittel; und
(d) gegebenenfalls $C_{2-6}$-Alkanoylieren der 23-Hydroxygruppe.

6. Verbindung, erhältlich durch Umsetzung von C-076 B1a auf folgende Weise:

(a) Entfernen der α-*L*-Oleandrosyl-α-*L*-oleandrosegruppe durch Hydrolyse;
(b) Ersetzen der in Stufe (a) erhaltenen 13-Hydroxygruppe durch eine 13-Halogengruppe durch Umsetzung mit einem ausreichend reaktiven Benzolsulfonylhalogenid in Gegenwart einer Base;
(c) gegebenenfalls Entfernen der 13-Halogengruppe mit einem selektiven Reduktionsmittel;
(d) gegebenenfalls Reduzieren der 22,23-Doppelbindung zu einer Einfachbindung durch Hydrieren unter Verwendung eines Lösungsmittels und eines Katalysators der in Stufe (d) von Anspruch 1 angegebenen Formel;
(e) gegebenenfalls $C_{2-6}$-Alkanoylieren der 5-Hydroxygruppe;
(f) gegebenenfalls Herstellen eines 23-($C_{1-6}$-Alkoxy)- oder 23-($C_{1-6}$-Alkylthio)-derivats durch Umsetzung mit einem $C_{1-6}$-Alkanol oder $C_{1-6}$-Alkylthiol in Gegenwart einer Säure; und
(g) gegebenenfalls Oxidieren eines in Stufe (f) erhaltenen 23-($C_{1-6}$-Alkylthio)-derivats in eine $C_{1-6}$-Alkylsulfinyl- oder $C_{1-6}$-Alkylsulfonylgruppe.

7. Verbindung, erhältlich durch Umsetzung von C-076 B1b auf folgende Weise:

(a) Entfernen der α-*L*-Oleandrosyl-α-*L*-Oleandrosegruppe durch Hydrolyse;
(b) Ersetzen der in Stufe (a) erhaltenen 13-Hydroxygruppe durch eine 13-Halogengruppe durch Umsetzung mit einem ausreichend reaktiven Benzolsulfonylhalogenid in Gegenwart einer Base;
(c) gegebenefalls Entfernen der 13-Halogengruppe mit einem selektiven Reduktionsmittel;
(d) gegebenenfalls Reduzieren der 22,23-Doppelbindung zu einer Einfachbindung durch Hydrieren unter Verwendung eines Lösungsmittels und eines Katalysators der in Stufe (d) von Anspruch 1 angegebenen Formel;
(e) gegebenenfalls $C_{2-6}$-Alkanoylieren der 5-Hydroxygruppe;
(f) gegebenenfalls Herstellen eines 23-($C_{1-6}$-Alkoxy) oder 23-($C_{1-6}$-Alkylthio)-derivats durch Umsetzung mit einem $C_{1-6}$-Alkanol oder $C_{1-6}$-Alkylthiol in Gegenwart einer Säure; und
(g) gegebenenfalls Oxidieren eines in Stufe (f) erhaltenen 23-($C_{1-6}$-Alkylthio)-derivats in eine $C_{1-6}$-Alkylsulfinyl- oder $C_{1-6}$-Alkylsulfonylgruppe.

8. Verbindung, erhältlich durch Umsetzung von C-076 B2a auf folgende Weise:

(a) Entfernen der α-*L*-Oleandrosyl-α-L-oleandrosegruppe durch Hydrolyse;
(b) Ersetzen der in Stufe (a) erhaltenen 13-Hydroxygruppe durch eine 13-Halogengruppe durch Umsetzung mit einem ausreichend reaktiven Benzolsulfonylhalogenid in Gegenwart einer Base;
(c) gegebenenfalls Entfernen der 13-Halogengruppe mit einem selektiven Reduktionsmittel; und
(d) gegebenenfalls $C_{2-6}$-Alkanoylieren einer oder beider der 5- und 23-Hydroxygruppen.

9. Verbindung, erhältlich durch Umsetzung von C76 B2b auf folgende Weise:

(a) Entfernen der α-*L*-Oleandrosyl-α-*L*-oleandrosegruppe durch Hydrolyse;
(b) Ersetzen der in Stufe (a) erhaltenen 13-Hydroxygruppe durch eine 13-Halogengruppe durch Umsetzung mit einem ausreichend reaktiven Benzolsulfonylhalogenid in Gegenwart einer Base;
(c) gegebenenfalls Entfernen der 13-Halogengruppe mit einem selektiven Reduktionsmittel; und
(d) gegebenenfalls $C_{2-6}$-Alkanoylieren einer oder beider der 5- und 23-Hydroxygruppen.

10. Verbindung, erhältlich durch Umsetzung von C-076 B1b auf folgende Weise:

(a) Entfernen der α-*L*-Oleandrosyl-α-*L*-oleandrosegruppe durch Hydrolyse;
(b) Ersetzen der in Stufe (a) erhaltenen 13-Hydroxygruppe durch eine 13-Halogengruppe durch Umsetzung mit einem ausreichend reaktiven Benzolsulfonylhalogenid in Gegenwart einer Base;
(c) Entfernen der 13-Halogengruppe mit einem selektiven Reduktionsmittel; und
(d) Reduzieren der 22,23-Doppelbindung zu einer Einfachbindung durch Hydrieren unter Verwendung eines Lösungsmittels und eines Katalysators der in Stufe (d) von Anspruch 1 angegebenen Formel.

11. Verbindung nach Anspruch 1 sur Verwendung bei der Behandlung von Parasiteninfektionen.

12. Verfahren zur Herstellung einer Verbindung nach Anspruch 1, in dem die Stufe (c) durch Behandlung der 13-Halogenverbindung mit einem Trialkylzinnhydrid in Gegenwart eines freiradikalischen Initiators durchgeführt wird.